# NEW EUROPEAN PATENT SPECIFICATION

(11) **EP 0 457 824 B2**
(45) Date of publication and mention of the opposition decision: **09.06.2004**
(45) Mention of the grant of the patent: 23.06.1999
(21) Application number: 90903297.1
(22) Date of filing: 13.02.1990
(51) Int. Cl.: C12Q 1/68, G01N 33/53

(54) **DETECTION OF A NUCLEIC ACID SEQUENCE OR A CHANGE THEREIN**
NACHWEIS EINER NUKLEINSÄURESEQUENZ ODER EINER ÄNDERUNG DARIN
DETECTION D'UNE SEQUENCE D'ACIDES NUCLEIQUES OU D'UN CHANGEMENT DANS CELLE-CI

(30) Priority: 13.02.1989 AU 270389
(43) Date of publication of application: 27.11.1991
(73) Proprietor: GeneCo Pty. Ltd., Brisbane, QLD 4000 (AU)
(72) Inventor: DALE, James, Langham, Moggill, QLD 4070 (AU); TIMMS, Peter, East Ipswich, QLD 4305 (AU); WALSH, Terence, Patrick, Acacia Ridge, QLD 4110 (AU)
(74) Representative: Campbell, Patrick John Henry
(86) International application number: PCT/AU1990/000058
(87) International publication number: WO 1990/009455

(56) References cited:
- EP-A- 123 513
- EP-A- 297 379
- WO-A-89/10414
- WO-A-90/01069
- WO-A-90/06042
- AU-A- 1 193 788
- AU-A- 3 058 589
- AU-B- 6 236 486
- US-A- 4 307 189
- US-A- 4 851 331
- Methods in Enzymol.,vol. 65,p. 560-580,1980 (A.J.H. Smith)

## Description

### Technical field

This invention relates to a method for detecting whether a specific nucleotide or base is at a particular position in a specific polynucleotide sequence, a method for detecting whether the same or different specific nucleotides or bases are at particular positions in at least two different specific polynucleotide sequences in a material having a plurality of different polynucleotide sequences, and screening methods for detecting whether the same or different specific nucleotides or bases are at particular positions in at least two different specific polynucleotide sequences in material having a plurality of different polynucleotide sequences.

### Background Art

At present, the most common technique for the detection of polynucleotide sequences is hybridisation using oligonucleotide or polynucleotide probes. For this technique, the target nucleic acid is usually bound, irreversibly, to a solid support such as cellulose or nylon. The labelled probe is then added in solution under hybridising conditions and if there is sequence homology between the probe and the target nucleic acid, the probe will form a hybrid molecule with the target polynucleotide. This hybrid can be detected in a variety of ways such as by radiolabelling or biotin labelling. The disadvantages of this technique are, firstly, that it requires considerable operator expertise, secondly, the technique is lengthy and time-consuming and thirdly, cannot be easily automated. Often the entire procedure can take more than 48 hours.

The liquid-solid methods normally employed for detecting specific nucleic acids in samples include Southern blot, Northern blot and dot blot hybridisations. These methods are slow, inefficient, technically demanding and are not easily automated. The Southern and Northern blot protocols have the further disadvantage of inefficient transfer of nucleic acid from gel to paper. Other groups have used liquid-solid hybridisations in which a capture probe is bound to a solid support and the DNA sequence of interest in the sample becomes bound to the capture probe. An example of this is in Australian Patent specification no. AU-70152/87 which describes using at least two oligonucleotide probes, which are complementary to mutually exclusive regions of the same target nucleic acid in a liquid hybridisation format to anneal the probes to the target, if it is present, and detecting the presence of the target by immobilisation of the two-probe target sandwich by one of the probes and subsequent detection of the other probe. However, this method requires a second, detector probe to hybridise to the DNA sequence of interest. This step reduces the specificity of the assay and subsequently increases background. The present invention overcomes this problem by involving only one extended primer which contains both capture and detection elements.

In contrast to liquid - solid hybridisation, liquid-liquid hybridisation has very rapid kinetics and improved sensitivity due to greater access of the probe to the target sequence. For example, Gene-Probe Inc uses a liquid hybridisation hydroxapatite method to detect DNA sequences. The main disadvantage of this system is that it relies on adsorptive discrimination of double-stranded DNA from single-stranded DNA sequences rather than sequence-specific separation of hybrid from excess probe. The present invention overcomes these disadvantages by allowing the nucleic acid hybridisations to occur in solution followed by the removal of the "hybrid" molecules onto a solid support matrix. Another potential advantage of liquid hybridisation is that a generalised solid support can work for a multitude of targets if the support - binding probes are labelled with the same capture molecule.

Several cases exist (Australian Patent specification nos. AU-A-70152/87, AU-A-26755/88, AU-A-53105/86, AU-B-89575/82 and AU-A-80097/87) which use a combination of two oligonucleotide probes to detect specific nucleic acid sequences in a sample. All these require the use of two short sequences of DNA on the target. These sequences must both be conserved in all possible target, must be mutually exclusive and non-overlapping and must have a similar G+C ratio to enable both probes to hybridise to their complementary sequence under the same conditions.

There is related background art concerning the use of a capture probe to detect the nucleic acid sequence of interest and to remove it from solution by binding the hybrid to a solid support matrix (Australian Patent specification nos. AU-A-70152/87, AU-A-53105/86, AU-A-21781/88, AU-A-69724/87, AU-A-14005/88). However, these techniques use separate capture and detector probes, resulting in a number of disadvantages as detailed above. The present invention overcomes these problems by using a single extended primer which incorporates both capture and detection elements.

There are many examples available of attaching non-radioactive reporter molecules to DNA, to enable the detection of specific hybrids. However, when biotin is incorporated into the DNA molecule for detection, even though several biotin molecules may be incorporated per target molecule (thereby increasing the sensitivity of detection) the mechanism of visualising the incorporated biotin is complex and time consuming.

By contrast, the incorporation of other non-radioactive reporter molecules (such as fluorescent, luminescent, chemiluminescent molecules) enables rapid and simple detection of the target sequence. However, the present art only enables a single reporter molecule to be attached to each target sequence. This fact reduces the overall sensitivity of the final assay.

There is, therefore, a demand for a simple method which utilises the rapid kinetics of liquid hybridisation, which only requires a single probe for analysis, and which results in stable hybrids thereby allowing the unhybridised material to be easily removed from the sequences to be detected. Accordingly, the present invention provides a liquid hybridisation system in which a single probe hybridises to the sequence of interest and is then covalently extended to produce a stable hybrid. This hybrid is then captured on to a support matrix and subsequently washed to removed unhybridised material. The system described by the present invention is simple, rapid, sensitive, can be read visually or on a simple plate reader, and may be readily automated.

In this area of nucleic acid hybridisation there is a need to detect two broad types of diseases: infectious and genetic. In relation to infectious diseases, a number of DNA based systems have been described to detect diseases caused by bacteria such as Salmonella, Neisseria, parasitic organisms such as Chlamydia and Rickettsiae, viruses such as hepatitis B and protozoa such as Plasmodium. However, all of these suffer one or more of the disadvantages listed above.

In relation to genetic diseases which are characterised by a mutation (deletion, insertion, point mutation or translocation), the technology is less well developed. These types of diseases are currently diagnosed either by using restriction fragment length polymorphism (RFLP) analysis or by precise hybridisation of short oligonucleotide probes. RFLP detection requires that a restriction enzyme site is altered by the mutation and this is not always the case. In addition, RFLP analysis requires the use of Southern blot hybridisation for detection. The use of short oligonucleotide probes to detect point mutations also has several serious disadvantages. In particular, the hybridisation conditions must be precise to ensure that a single base-pair mismatch does not result in hybridisation. In practice, salt concentration and temperature determine the specificity of the hybridisation conditions and these are not easily controlled to the required preciseness. The present invention overcomes the need for Southern hybridisation analysis and for precise control of hybridisation conditions. It achieves this by the specific primer hybridising to a constant section of the gene adjacent to the mutation and allowing the enzyme, a DNA polymerase, to extend the DNA chain up to and including the nucleotide or base mutation. By manipulation of the dideoxynucleotide added to the polymerase reaction all of the possible nucleotide changes can be detected.

Detection of both infectious and genetic diseases requires the incorporation of some type of labelled molecule into the system. Various radioactive isotopes or radioactivity labelled compounds may be used as labels. However, radioactive labels have several disadvantages, including; (i) hazardous, (ii) expensive, (iii) limited shelf life, (iv) require expensive equipment for measuring the signal generated. More recently, a range of non-radioactive substances have been used to detect DNA molecules. Examples of non-radioactive labels are fluorescent, luminescent, chemiluminescent, enzymatic or immunological compounds. Labels based on the affinity of biotin and avidin or streptavidin, lanthanide chelates, lectins and proteins may also be used. The preferred detection means would be by spectroscopy or photochemistry or by the formation of a detectable complex between the label moiety and the polypeptide, lectin, or antibody linked to an entity capable of generating a detectable change, including enzymes such as; alkaline phosphatase or horseradish peroxidase.

EP-A-0297379 describes a method for amplifying specific target nucleic acids involving use of immobilized primers. WO 89/10414 describes a process for the creation of new genetic markers and the simultaneous determination of multiple genetic markers comprising amplifying a genomic DNA sample spanning a sequence variation, detecting DNA sequence variation within the amplified fragment, and detecting the amplified fragment. US Patent No. 4,851,331 describes a probe polynucleotide which binds to a target nucleotide sequence in the nucleic acid of a biological sample, and then is enzymatically extended in the 3'-direction with a mixture of nucleoside triphosphates including at least one nucleoside triphosphate that has been detectably labelled. WO 90/06042 describes a method for detecting a target RNA or DNA in an analyte sample, which involves contacting the analyte with magnetic particles carrying a single stranded 5'-attached DNA probe capable of binding to said RNA or DNA.

However, what is lacking in the current technology is a single system which encompasses: (i) a rapid means of detecting a nucleotide in a polynucleotide sequence, (ii) which is sufficiently sensitive to detect low numbers of the target nucleotide in the sample and (iii) which is nonradioactive. At present, no single system satisfies all these requirements.

As a final aspect, the need to detect specific nucleotides in sequences in a sample requires the organisation of all steps either: (i) into a simple kit format, or (ii) into an automated device. Whereas both, kits and automated machines, are available for detecting proteins by way of antibodies, no systems are yet available which simply, rapidly and inexpensively detect specific nucleotides in polynucleotide sequences in a sample.

### Objects of Invention

An object is to provide methods for detecting whether a specific nucleotide or base is at a particular position in a specific polynucleotide sequence.

Another object is to provide methods for detecting whether the same or different specific nucleotides or bases are at particular positions in at least two different specific polynucleotide sequences in material having a plurality of different polynucleotide sequences.

Another object is to provide screening methods for detecting whether the same or different specific nucleotides or bases are at particular positions in at least two different specific polynucleotide sequences in material having a plurality of different polynucleotide sequences.

### Disclosure of Invention

The following abbreviations and definitions apply throughout the specification and claims:
- SP -: specific primer: a nucleotide sequence complementary to a portion of the target sequence
- Target -: the nucleotide sequence to be detected in the sample; can be derived from, for example, any infectious or parasitic organism including any virus, fungus, bacteria, mycoplasma, nematode, protozoan etc
- Polymerase Enzyme -: any DNA dependent DNA polymerase or RNA dependent DNA polymerase
- dNTP -: all four deoxyribonucleotides that is dATP, dCTP, dGTP and dTTP as well as dITP and dUTP
- ddNTP -: all four dideoxyribonucleotides that is ddATP, ddCTP, ddGTP and ddTTP as well as ddITP
- D -: detector molecule: a molecule which can be covalently attached to a nucleotide or nucleotide sequence and that can be subsequently assayed for either directly or indirectly. For example, biotin; radioisotopes of carbon, hydrogen, iodine, phosphorus and sulphur; any antigen; haptens; fluorescent molecules including fluorescein, rhodamine and eosin; enzymes including alkaline phosphates, peroxidases and luciferase; any monoclonal antibody
- dNTP-D -: a detector molecule covalently bound to one of the four deoxyribonucleotides
- C -: capture molecule: a molecule which can be covalently attached to a nucleotide or nucleotide sequence and that will subsequently bind to a specific affinity molecule. For example, biotin (binds with avidin or streptavidin); antibody (binds with antigen); antigen (binds with antibody)
- SAM -: specific affinity molecule: a molecule that will bind specifically with a particular capture molecule. For example, avidin or streptavidin; an antibody; an antigen
- SS -: solid support: any solid matrix to which the SAM is attached. For example, agarose; polyacrylamide; magnetic beads; polystyrene; microtitre plates; nylon; nitrocellulose
- Wash -: addition and removal of a solution for the purpose of removing unreacted molecules
- Stringency -: conditions of salt and temperature which affect the stability of the hydrogen bonds between two nucleic acid molecules. For example, the hydrogen bonds are most unstable at high stringency reflecting either high temperature or low salt or both.
- Assay -: any procedure that is specific for detecting the detector molecule and that can be measured either qualitatively or quantitatively
- X -: any one of the four deoxyribonucleotides
- Y -: any one of the four ribonucleotides or deoxyribonucleotides; in the example Y will form hydrogen bonds with X, ie, a nucleotide sequence of nine Y's will be complementary to a nucleotide sequence of 9 X's
- Z -: any one of the four nucleotides forming a sequence not complementary to the sequence
- ddT -: dideoxythymidine-5'-triphosphate: ddT has been used as example as it base pairs with A in the target sequence. If the base to be detected was
- ddT-C -: C then ddG would be used, G with ddC and T with ddA. dideoxythymidine-5'-triphosphate/capture molecule complex: ddT has been used as example as it base pairs with A in the target sequence. If the base to be detected was C then ddG would be used, G with ddC and T with ddA. The capture molecule is covalently attached to ddT and represents any one of the capture molecules described above.
- immediately adjacent -: means that there are no nucleotides or bases between a primer bound to part of a specific polynucleotide sequence and a specific nucleotide or base to be detected
- fraction -: means at least a portion of a mixture resulting from the reaction of an oligonucleotide primer(s), extending reagents, specific polynucleotide sequence(s), detectable element(s) and/or separation elements
- intervening sequence -: means at least one nucleotide or base between a primer bound to part of a specific polynucleotide sequence and a specific nucleotide or base to be detected
- oligonucleotide primer -: a single stranded nucleic acid molecule with a typical length of 5 to 60 nucleotides but can be 200 or more nucleotides which has a sequence complementary to part of the polynucleotide sequence to be detected
- specific polynucleotide sequence -: a partly or completely known sequence of nucleotides
- hybridisation -: the physical association of the oligonucleotide primer with the complementary region of the target polynucleotide sequence to form a double stranded hybrid nucleic acid molecule

An interfering detectable and/or separation element is, for instance, one which is the same as that which is incorporated at the position complementary to the specific nucleotide or base to be detected, eg:
(A) Consider a single nucleotide or base N₁ to be detected in a specific polynucleotide sequence S₁. Assume there is a bound oligonucleotide primer P₁ having a sequence complementary to part of S₁ and which is bound to S₁. Assume there are intervening nucleotides N_{A}, N_{B} and N_{C} between P₁ and N₁. Extend P₁ up to and including N₁ with complementary nucleotides to N_{A}, N_{B}, N_{C} and N₁, namely, N_{D}, N_{E}, N_{F} and N₂-S₁ (S₁ corresponding to a separation element) respectively; then the following conditions are required:
   N_{A} N_{B} or N_{C} cannot be N₁
(B) Consider two nucleotides or bases N₁ and N₂ to be detected in two different specific polynucleotide sequences S₁ and S₂. Assume there are two different oligonucleotide primers P₁ and P₂ (bound to S₁ and S₂ respectively) having sequence complementary to part of S₁ and S₂.

Assume there are intervening nucleotides N_{A}, N_{B} and N_{C} between P₁ and N₁ and intervening nucleotides X_{X}, N_{Y} and N_{Z} between P₂ and N₂. Extend (a) P₁ up to and including N₁ with complementary nucleotides to N_{A}, N_{B}, N_{C} and N₁, namely, N_{D}, N_{E}, N_{F} and N₃-D₁ (D₁ corresponding to a detectable element) respectively; and (b) P₂ up to and including N₂ with complementary nucleotides to N_{X}, N_{Y}, N_{Z} and N₂, namely, N_{O}, N_{P}, N_{Q} and N₄-D₂ (D₂ corresponding to another detectable element);
then the following conditions are required:

If N1= N2 and D1 = D2
then N_A )
N_B )
N_C ) cannot be N1
N_X )
N_Y )
N_Z )

If N1 does not equal N2 D1 = D2
then N_A )
N_B )
N_C ) cannot be N1 or N2
N_X )
N_Y )
N_Z )
If N1 does not equal N2 D1 does not equal D2
N_A )
N_B ) cannot be N1
N_C )

N_x )
N_Y ) cannot be N2
N_z )

According to the invention, there is provided a method for detecting whether a specific nucleotide or base is at a particular position in a specific polynucleotide sequence in material comprising:
a) exposing, under hybridizing conditions, said material to an oligonucleotide primer having a sequence complementary to part of the specific polynucleotide sequence wherein said primer incorporates a separation element or a detectable element at the 5' end of said primer and wherein said primer binds to part of said specific polynucleotide sequence in said material (i) immediately adjacent to the particular position or (ii) not immediately adjacent to the particular position whereby there is an intervening sequence between the particular position and primer bound to the specific polynucleotide sequence;
b) extending primer bound to the specific polynucleotide sequence with the proviso that the bound primer is only extended up to and including said specific nucleotide or base when said specific nucleotide or base is at the particular position in the specific polynucleotide sequence wherein the extended primer has a detectable element and/or a separation element at a position complementary to said specific nucleotide or base with the proviso that said extended primer has at least one separation element and at least one detectable element and, when there is an intervening sequence between the particular position and primer bound to the specific polynucleotide sequence, with the further proviso that the intervening sequence cannot be one where bases or nucleotides complementary to the intervening sequence and which are incorporated into the extended primer cause incorporation of an interfering detectable and/or separation element;
c) separating any extended primer into a fraction wherein said fraction does not have detectable element not included in said extended primer with the proviso that said separating does not include the step of digesting the specific polynucleotide sequence having said extended primer bound thereto; and
d) determining whether said extended primer is present in said fraction by assaying said fraction for said extended primer wherein the presence of said extended primer indicates that the specific nucleotide or base is at the particular position in the specific polynucleotide sequence and the absence of said extended primer indicates that the specific nucleotide or base is not at the particular position in the specific polynucleotide sequence;
wherein said extending comprises using at least one dideoxynucleotide, said dideoxynucleotide being complementary to said specific nucleotide or base.

The invention also provides a method for detecting whether the same or different specific nucleotides or bases are at particular positions in at least two different specific polynucleotide sequences in material having a plurality of different polynucleotide sequences comprising:
a) exposing, under hybridizing conditions, said material to at least two different oligonucleotide primers each of said different oligonucleotide primers having a sequence complementary to part of one of said different specific polynucleotide sequences wherein:
   each of said primers binds to its complementary polynucleotide sequence when present in said material, each of said oligonucleotide primers binding to part of a different specific polynucleotide sequence to that of the other primer(s),
   each of said primers binds to its complementary specific polynucleotide sequence in said material (i) immediately adjacent to the particular position or (ii) not immediately adjacent to the particular position whereby there is an intervening sequence between the particular position and primer bound to the specific polynucleotide sequence when present in said material, and
   each of said primers incorporates a separation element or a detectable element at the 5' end of said primer;
b) extending said different oligonucleotide primers bound to their complementary polynucleotide sequences with the proviso that each of the bound primers is only extended up to and including the particular position when said specific nucleotide or base is at the particular position in the specific nucleotide sequence wherein each of said extended primers has a detectable element and/or a separation element at a position complementary to said specific nucleotide or base, with the further proviso that each of said extended primers has at least one separation element and at least one detectable element and, when there are intervening sequences between the particular positions and primers bound to the specific polynucleotide sequences, with the further proviso that the intervening sequences cannot be ones where bases or nucleotides complementary to the intervening sequences and which are incorporated into the extended primer(s) cause incorporation of interfering detectable and/or separation element(s);
c) separating any extended primer(s) which has extended only up to a specific nucleotide or base at a particular position into at least one fraction wherein said fraction does not have detectable element not included in said extended primer with the proviso said separating does not include the step of digesting the specific polynucleotide sequence having said extended primer bound thereto; and
d) determining whether any of said extended primers are present in said fraction(s) by assaying said fraction(s) for said extended primer(s) wherein the presence of an extended primer indicates that a specific nucleotide or base is at a particular position in a specific polynucleotide sequence and the absence of said extended primer(s) in a fraction(s) indicates that at least one specific nucleotide or base is not at a particular position in at least one different specific polynucleotide sequence;
wherein said extending comprises using at least one dideoxynucleotide, said dideoxynucleotide being complementary to said specific nucleotide or base.

The invention also provides a screening method similar to the above method for detecting whether the same or different specific nucleotides or bases are at particular positions in at least two different specific polynucleotide sequences in material having a plurality of different polynucleotide sequences.

Generally the method uses:
a nucleic acid primer specific to part of a partly or wholly known nucleotide sequence which is immediately adjacent to the nucleotide or base to be detected or specific to part of a partly or wholly known nucleotide sequence which is not immediately adjacent to the nucleotide or base to be detected but has an intervening sequence between the bound primer and the nucleotide or base to be detected;
extension of the primer catalysed by a nucleic acid polymerase enzyme;
either (i) the attachment of a capture molecule at the 5'-end of the specific primer, addition of the target sequence under hybridisation conditions and the incorporation of a detector molecule(s) in the enzyme catalysed extended primer or (ii) the attachment of a detector molecule at the 5'-end of the specific primer, addition of the target sequence under hybridisation conditions and the incorporation of a capture molecule(s) in the enzyme catalysed extended primer;
capture of the extended primer using a specific affinity molecule attached to a solid support; and
assay for the detector molecule.

Advantageously, extending the primer comprises using four different nucleotides selected from the group consisting of dATP, dCTP, dGTP, dTTP, dUTP, dITP, ddATP, ddCTP, ddGTP, ddITP, and ddTTP, at least one of said nucleotides being selected from the group consisting of ddATP, ddCTP, ddGTP, ddITP and ddTTP, said nucleotides incorporating a detectable element and/or a separation element.

The extending may comprise using one nucleotide selected from the group consisting of ddITP, ddATP, ddCTP, ddGTP, and ddTTP, said nucleotide incorporating a detectable element and/or a separation element.

In addition to the nucleotide(s) also typically used in extending the primer are at least one appropriate polymerase enzyme and appropriate buffering agent.

Examples of polymerase enzymes are E.coli DNA polymerase, E.coli DNA polymerase (Klenow fragment), Bacteriophage T7 and DNA polymerase, Bacteriophage T4 DNA polymerase, Taq DNA polymerase and AMV Reverse transcriptase.

Buffering agents which buffer aqueous solutions between pH 6 - 8.5 are generally suitable for use with a polymerase enzyme to extend the primer. Generally a magnesium salt (eg MgCl₂ or MgSO₄) is included with the buffering agent. A total salt concentration between 50 and 300 mM is generally acceptable.

Temperature of the extending reaction is chosen according to the polymerase enzyme used and is typically in the range 25-80°C, more typically 30-70°C.

It is preferred that at least one of the nucleotides has the detectable element.

Alternatively, or in addition, the detectable element is linked to the oligonucleotide primer.

Typically, the detectable element is a radioactive label selected from the group consisting of ³H, ¹²⁵I, ¹³¹I, ¹⁴C, ³⁵S and ³²P.

The detectable element may be non- radioactive and generally is selected from the group consisting of an enzymatic group, an immunological group, and a spectroscopically detectable group. The spectroscopically detectable group may be a luminescent group, a chemiluminescent group, an NMR detectable group, a fluorescent group or an 1 R detectable group.

Generally, the specific polynucleotide sequence is a DNA sequence or an RNA sequence.

The specific polynucleotide sequence may be from an infectious or disease causing organism which may be live or non-viable viral, bacterial, fungal or Protozoan.

The extending may include:
adding a plurality of nucleotide types to said primer bound to the polynucleotide sequence to extend said primer; or adding a single nucleotide type to said primer bound to the polynucleotide sequence to extend said primer; or
adding at least one single nucleotide type to said primer bound to the polynucleotide sequence to extend said primer.

In one form of this invention, the separation element is linked to the oligonucleotide primer. Alternatively, the extended primer includes a separation element which facilitates separation of extended primer from the mixture and wherein at least one of said nucleotides has the separation element.

In one form of the invention, the separation step comprises:
contacting any extended primer with a molecule having affinity for the separation element, the molecule being linked to a support that facilitates the separating; and separating the contacted extended primer(s) to provide the fraction.

Advantageously, the separation element and the molecule are selected from the group listed immediately below:

| Separation Element | | Molecule Having Affinity for Separation Element |
|---|---|---|
| (a) | a ligand for which there is a receptor; | a receptor for the ligand; |
| (b) | a receptor; | the ligand for the receptor; |
| (c) | an antigen; | an antibody for the antigen; |
| (d) | an antibody for an antigen; | the antigen; |
| (e) | an antiidiotypic antibody; | an antibody for the antiidiotypic antibody; |
| (f) | an antibody for an antiidiotypic antibody; | an antiidiotypic antibody for the antibody; |
| (g) | a haptenic group; | an antibody for the haptenic group; |
| (h) | an antibody for a haptenic group; | the haptenic group; |
| (i) | an enzyme; | a binding inhibitor for the enzyme; and |
| (j) | a binding inhibitor for an enzyme; | the enzyme. |

Typically, the separation element and the molecule are selected from the group listed below:

| Separation Element | | Molecule Having Affinity for Separation Element |
|---|---|---|
| (a) | a ligand for which there is a specific receptor; | a specific receptor for the ligand; |
| (b) | a specific receptor; | the ligand for the specific receptor; |
| (c) | an antigen; | a specific antibody for the antigen; |
| (d) | a specific antibody for an antigen; | the antigen; |
| (e) | an antiidiotypic antibody; | an antibody specific for the antiidiotypic antibody; |
| (f) | an antibody for an antiidiotypic antibody; | an antiidiotypic antibody specific for the antibody; |
| (g) | a haptenic group; | a specific antibody for the haptenic group; |
| (h) | a specific antibody for a haptenic group; | the haptenic group; |
| (i) | an enzyme; | a tight binding inhibitor for the enzyme; and |
| (j) | a tight binding inhibitor for an enzyme; | the enzyme. |

Typical examples of ligands for which there are available receptors are:
a vitamin such as biotin, a sugar molecule such as glucose or mannose, a hormone such as adrenalin or cortisone and a steroid such as progesterone or testosterone. There are numerous types of ligands which have available receptors and the preceding list is given by way of exemplification only.

The separation typically includes a solid support which is typically selected from the group consisting of latex, magnetic beads, nitrocellulose, agarose, cellulose, polyacrylamide, nylon, glass and polystyrene.

### Advantages

### 1. General Advantages

A particular advantage of the method of the invention is that the system combines separation and detection elements on the one oligonucleotide after primer extension. Most other systems require the use of a capture probe and a detector probe.

A further advantage is that the system depends on the enzyme driven extension of the primer to incorporate a separation or detection element or elements (depending on configuration) and the the element or elements are therefore covalently attached to the specific primer. Because of this covalent attachment, washing steps can be performed at very high stringency which results in reduced background and increased specificity.

Another advantage is that the method of the invention can be easily automated.

In the method of the invention the steps can be incorporated into a simple kit format.

A further advantage is that an oligonucleotide primer having a sequence complementary to a conserved or variable region of the genome of the organism of interest can be used and thus can be designed for high or low specificity (i.e., genus specific, species specific or strain specific). The invention provides a rapid and sensitive method for the detection of specific nucleotide changes in specific polynucleotide sequences using a single oligonucleotide probe.

Generally in the method of the invention the detectable hybrid is captured by a solid matrix to allow washing away of unincorporated detectable element. While this is not a difficult step it does add to the time required for processing. The step can be overcome by choosing a detectable element whereby any unincorporated detectable element can be simply inactivated and left in the reaction mix, rather than having to be physically removed.

Generally, a detection sensitivity in the order of 10³ - 10⁴ genome copies is quite adequate for detecting a wide range of virus, bacterial and parasitic organisms which are usually present in moderate to high numbers in their respective disease states. For defect diseases in which the infectious agent is present only in very low numbers (e.g. HIV) the method of the invention can make use of the technique of polymerisation chain reaction (PCR) which is effectively an amplification step which results in an increase in sensitivity.

### 2. Infectious Disease Diagnosis Advantages

The method of the invention permits the rapid, simple and non-hazardous detection of specific nucleotides in specific polynucleotide sequences in samples of biological material especially infectious agents. The sequence may be part of an infectious organism such as a virus, bacterium, Protozoan or fungus, or part of a gene in which an abnormality results in a genetic disorder: for example, sickle-cell anemia, cystic fibrosis, α-thalassemia or β-thalassemia.

Non-viable organisms can be detected using the method of the invention.

A further advantage is the method can use a mixture of oligonucleotide primers for the detection of a battery of agents suspected of causing a broad range of disease states (e.g., pneumonia; mycoplasma, chlamydia, streptococcus, legionella, RSV).

### 3. Genetic Disease Diagnosis Advantages

The method can be used for the rapid detection of an altered nucleotide base (point mutation) and for the detection of the insertion or deletion of one or more nucleotides in a polynucleotide sequence. In this instance the genetic change has to be known and characterised at the DNA sequence level.

The detection of a single or more base changes can be automated.

The method can be adapted to large scale screening for single (or more) nucleotide changes. This is particularly important in screening for genetic diseases such as cystic fibrosis but can also be adapted to the differentiation of alleles not necessarily involved in gene expression such as used for DNA profiling.

This particular method does not involve solid-liquid hybridisation, precise liquid hybridisation conditions and is not technically demanding. Other systems, such as Southern blot hybridisation, require that the target polynucleotide sequence is linked to a solid support (technically demanding) and/or the use of oligonucleotide probes that require precise hybridisation conditions. These systems cannot be automated and are not easily adapted to large scale screening.

### Brief Description of Drawings

Preferred embodiments of the invention are now described with reference to the following drawings in which:
Figure 1 is a schematic drawing of a method for detecting a specific polynucleotide sequence.
Figure 2a is a schematic drawing of a method for detecting a specific polynucleotide sequence using a separation element which links to the oligonucleotide primer portion of an extended primer;
Figure 2b is a schematic drawing of a method for detecting a specific polynucleotide sequence using a separation element which links to the extended portion of an extended primer.
Figure 3a is a schematic drawing of a method according to the invention for detecting a nucleotide or base change using a separation element which links to the oligonucleotide primer portion of an extended primer;
Figure 3b is a schematic drawing of a method according to the invention for detecting a nucleotide or base change using a separation element which links to the extended portion of an extended primer;
Figure 3c is a schematic drawing of a method for detecting a nucleotide or base change using a detectable element linked to the extended portion of an extended primer.
Figure 4 shows an autoradiograph used to verify the size of the extended oligonucleotide produced in the third example; and
Figure 5 is a schematic depiction of an apparatus for performing any one of the methods of the invention.

### Best Mode and Other Modes of Carrying out the Invention

There are two especially preferred applications of the methods of the invention, that is, (i) the detection of a nucleotide or base change (major applications: genetic diseases and DNA fingerprinting) and (ii) detection of multiple nucleotide or base changes in different polynucleotide sequences (major applications: multiple genetic diseases and DNA fingerprinting). A single apparatus (with minor modifications) is suitable for use with both applications.

### 1. Detection of a nucleotide or base change

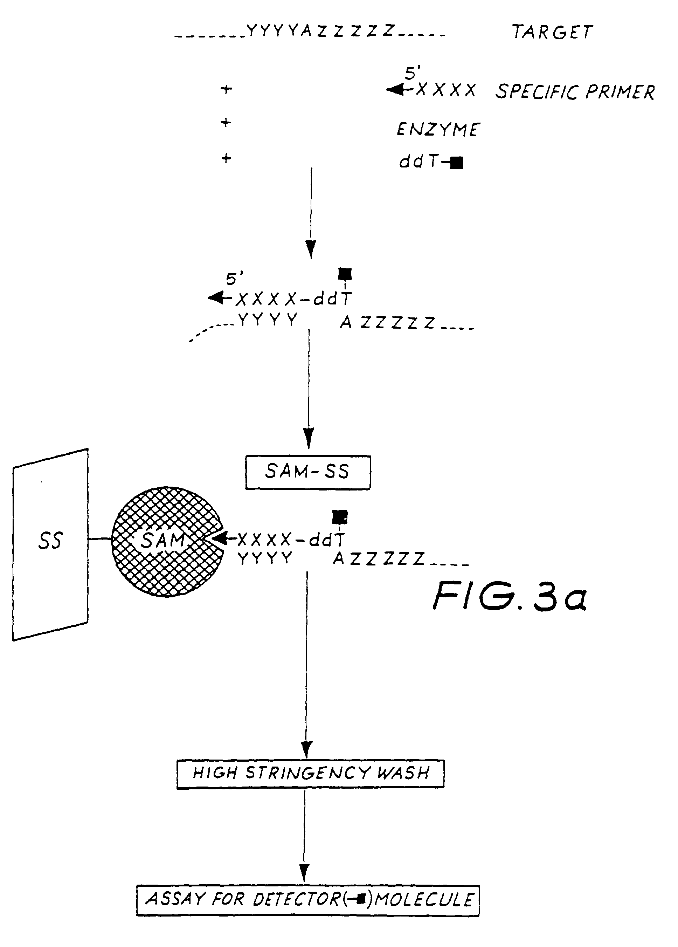

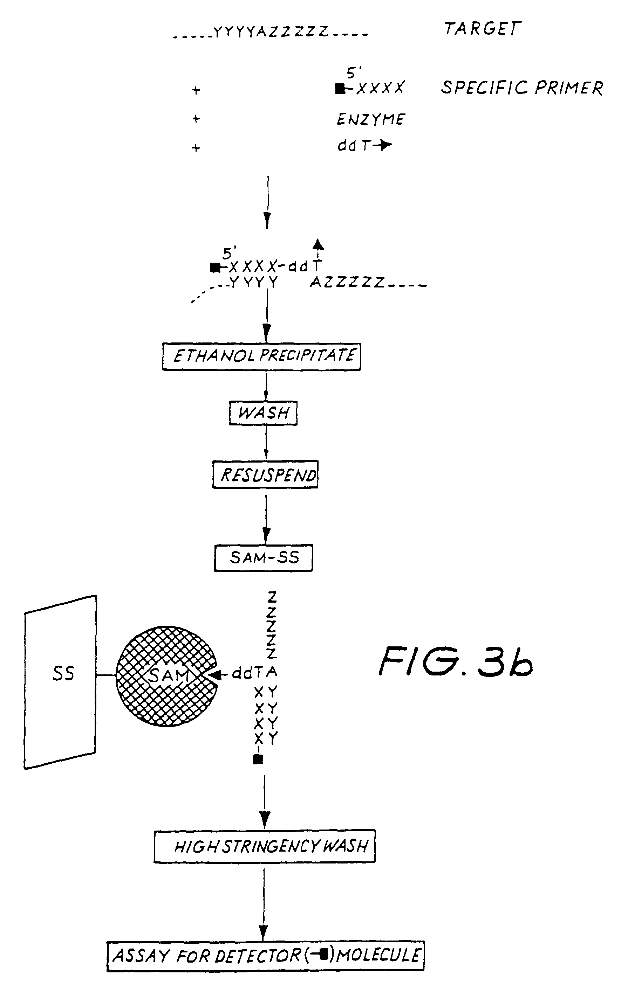

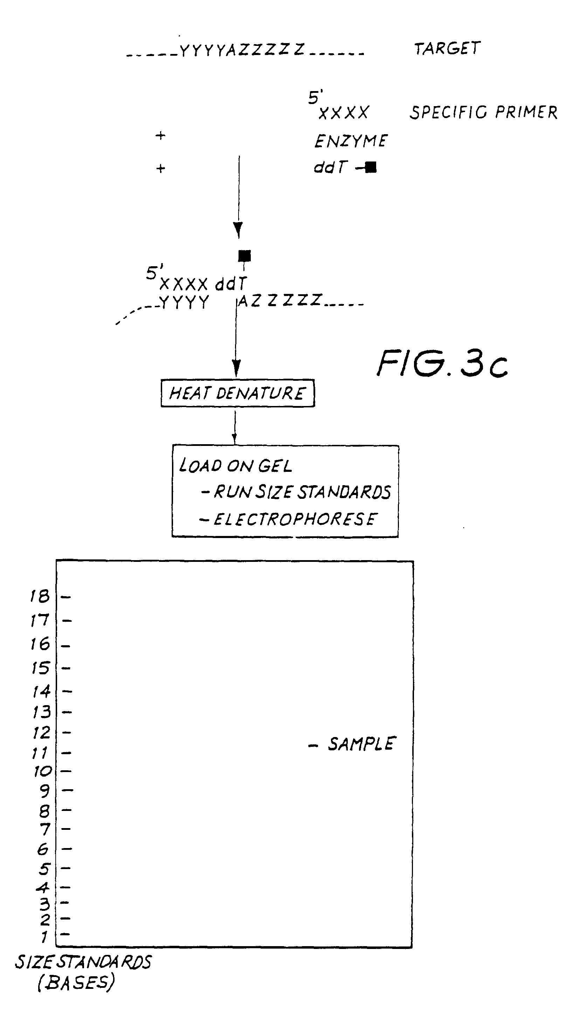

This method involves the following steps:
· The synthesis of a specific primer complementary to part of a known or partly known nucleic acid sequence, the target. The specific primer sequence is complementary to the target sequence either immediately adjacent to but not including the single nucleotide or base to be detected or not immediately adjacent to the single nucleotide or base to be detected but having an intervening sequence between the bound primer and the nucleotide or base to be detected
· The attachment of a capture (Configuration A - Figure 3a) or detector (Configuration B - Figure 3b) molecule to the 5'-end of the specific primer.
· The addition to the solution of only one type of dideoxyribonucleotide (or deoxyribonucleotide), that is, the one which can pair or hydrogen bond with the base or nucleotide to be detected in the target sequence and either a DNA dependent DNA polymerase (if the target is DNA) or a RNA dependent DNA polymerase (if the target is RNA) under conditions suitable for the enzymatic extension of the specific primer. The dideoxyribonucleotide (or deoxyribonucleotide) has covalently attached to it a detector molecule (Configuration A) or a capture molecule (Configuration B), The polymerase enzyme extends the primer using the target nucleic acid as a template adding only one molecule of the dideoxyribonucleotide (or deoxyribonucleotide) if base pairing is possible. The specific primer is not extended if base pairing between the dideoxyribonucleotide (or deoxyribonucleotide) and the target sequence is not possible.
· The reaction can be heat denatured and the cycle repeated to amplify the amount of extended specific primer.
· For Configuration A, the specific affinity molecule (attached to a solid support) is added to the solution under conditions conducive to the binding of the capture molecule to the specific affinity molecule
· For Configuration B, the extended specific primer is precipitated with ethanol, washed to remove unincorporated dideoxyribonnucleotide-capture molecule complex (or deoxyribonucleotide-capture molecule complex). After washing, the extended specific primer is resuspended in a solution conducive to the binding of the capture molecule to the specific affinity molecule. The specific affinity molecule (attached to a solid support) is then added
· For Configurations A and B, once the extended specific primer is attached to the specific affinity molecule-solid support complex via the capture molecule, the mix is washed extensively under high stringency conditions.
· For Configuration A and B, at the conclusion of the washing step, the mix is assayed using a procedure appropriate for detecting the detector molecule.

### 2. Detection of a multiple nucleotide or base chances in different polynucleotide sequences, ie multiple genetic defects or diseases or genetic fingerprinting

This method involves the following steps:
· The synthesis of multiple different specific primers each of which is complementary to a different known nucleic acid sequence, the targets. The different specific primer sequences are complementary to their respective different target sequences immediately adjacent to the single base to be detected,
· The attachment of different capture molecules to the 5'-end of the different specific primers
· The hybridization, in solution, of these different specific primers to their different respective target nucleic acid sequences
· The addition of the solution of all four types of dideoxyribonucleotide (eg, ddATP, ddCTP, ddGTP, ddTTP) and either a DNA dependent DNA polymerase (if all the targets are DNA) or a RNA dependent DNA polymerase (if all the targets are RNA) or both (if the targets are a mixture of both DNA and RNA) under conditions suitable for the enzymatic extension of the different specific primers. Each dideoxyribonucleotide has covalently attached to it a different detector molecule.
· The polymerase enzyme extends the different specific primers using the different target nucleic acid sequences as templates adding only one molecule of one of the dideoxyrlbonucleotides.
· The reaction can be heat denatured and the cycle repeated to amplify the amounts of extended different specific primers.
· The specific affinity molecules (with specific affinity for the different respective capture molecules and attached to solid supports such as "test strips") are added to the solution under conditions conducive to the binding of the different capture molecules to the different specific affinity molecules,
· Once the different extended specific primers are attached to their specific affinity molecule-solid support complexes via the different capture molecules, each different complex is removed individually with the appropriate test strip which subsequently is washed extensively under high stringency conditions.
· At the conclusion of the washing step, one of the test strips is assayed for the presence of detector molecule using a procedure appropriate for the particular detector molecule. If the assay indicates that the detector molecule is present on the test strip then this is indicative that the specific nucleotide sequence targeted by the primer having that particular detectable molecule is present in the test sample. If the assay indicates that no detector molecule is present on the test strip then this is indicative that the specific nucleotide sequence targeted by the primer having that particular detectable molecule is not present in the test sample.
· The procedure of the last step is repeated for each of the test strips

### 3. Apparatus for Carrying out Methods of the Invention

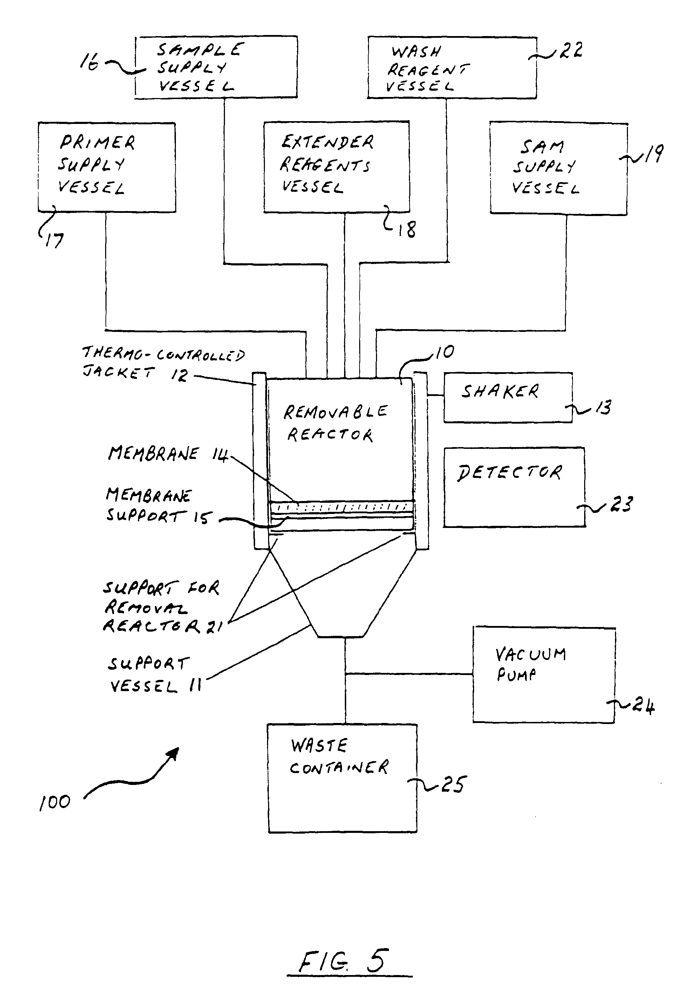

Referring to Fig. 5 apparatus 100 is generally suitable for carrying out the methods of the invention according to non-automatic or automatic procedures. The extending and separation procedures take place within removable reactor 10 which is disposed within support vessel 11. Vessel 11 is temperature controlled by thermo-controlled jacket 12. The contents of reactor 10 are mixed by shaking vessel 11 using shaker 13. The base of reactor 10 has specific porosity membrane 14 supported by membrane support 15. Reactor 10 is supported in vessel 11 by support for removable reactor 21.

Sample material, primer(s) and extending reagents are delivered to reactor 10 from their respective vessels 16, 17 and 18. Primer annealing and extension are permitted to occur for an appropriate period under gentle mixing and fixed temperature conditions. This reaction creates an extended primer having a detectable element and separation element provided the sample has a specific polynucleotide sequence for which the primer has a sequence complementary to part of the specific polynucleotide sequence.

Subsequently specific affinity molecule(s) (SAM(S)) attached to an inert support for the separation element are added to reactor 10 from SAM supply vessel 19 and allowed to react with extended primer for an appropriate period. Unreacted reagents, buffer and unextended primer(s) are then removed to waste container 25 via vacuum line 25, having associated vacuum pump 24, attached to the base of support vessel 11 whilst extended primer(s) having attached SAM(S) are retained in reactor 10 by membrane 14. The retained primer-SAM complexes are washed with wash reagent supplied from vessel 22 to remove background and thereby create the first fraction in reactor 10. The first fraction is then examined for the presence of detectable element with detector 23. Typically, the detectable element is a radioactive element such as ³²P and detector 23 is a scintillation counter.

If the assay indicates that the detector molecule is present then this is indicative that the specific nucleotide sequence is present in the test sample. If the assay indicates that no detector molecule is present then this is indicative that the specific nucleotide sequence is not present in the test sample.

### Example 1

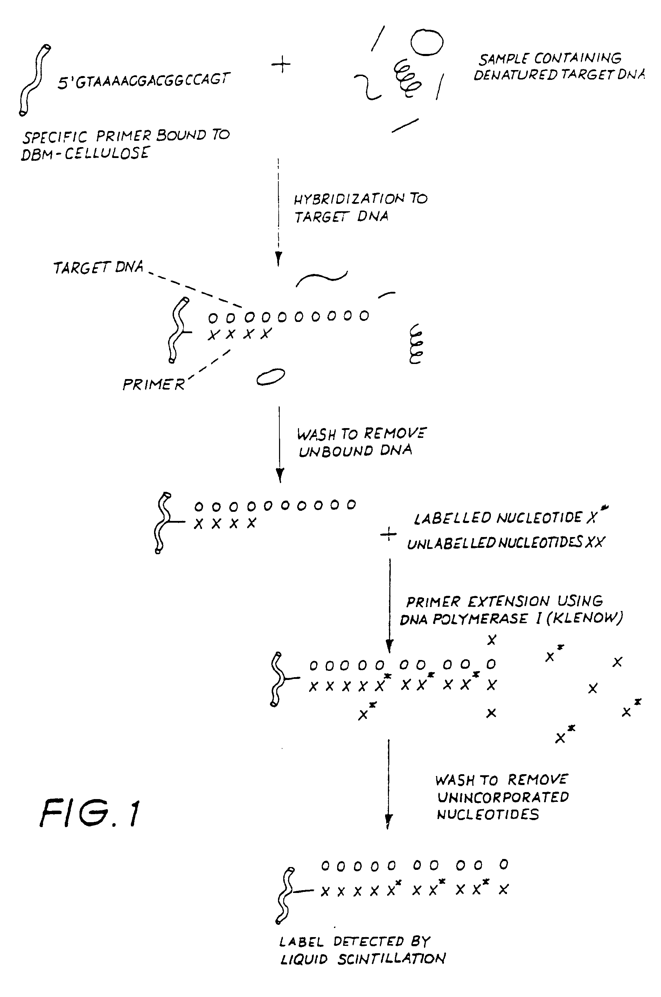

In this example of the detection of a specific polynucleotide sequence, the method depicted in Fig. 1 was used for the detection of the single stranded genomic DNA of the bacteriophage M13.

### 1. Synthesis of the synthetic primer.

A 17-mer DNA complementary to M13 ssDNA was synthesized on an Applied Biosystems DNA Synthesizer.

The 17-mer had the nucleotide sequence:

3'-T-G-A-C-C-G-G-C-A-G-C-A-A-A-A-T-G- 5'

This primer is not only complementary to M 13 single-stranded DNA but also to the bacterial double-stranded DNA plasmid, pUC12.

### 2. Attachment of the primer to cellulose.

The method of Ashley and MacDonald (1984) was used to attach the primer to ABM-cellulose. The amino benzyloxymethyl (ABM) groups on ABM cellulose were diazotized to form diazobenzyloxymethyl-cellulose (DBM-cellulose). The specific primer was attached to the DBM-cellulose. Approximately 40 µg of specific primer was bound to 20 mg of cellulose.

### 3. Hybridization of specific primer-cellulose (SP-cellulose) with target nucleic acid

SP-cellulose was resuspended in a solution containing the target nucleic acid either M13 ssDNA or pUC12 and water. This was mixed, boiled for 5 minutes and quenched on ice. The mixture was then washed by centrifugation using a wash buffer comprising 140mM sodium chloride, 15mM sodium citrate and 20mM sodium phosphate, pH 7.0 to remove nucleic acids that had not hybridised with the SP-cellulose.

### 4. Primer extension

The specific primer was extended using DNA polymerase I (Klenow fragment) together with labelled and non-labelled nucleotides.

The reaction conditions were as follows:

| 20 : 1 Reaction Mix | |
|---|---|
| 50mM | Tris-HCl, pH 8.3 |
| 8mM | Magnesium chloride |
| 4mM | dithiothreitol |
| 4mM | sodium pyrophosphate |
| 1mM | each dATP, dGTP and dTTP |
| 1 Ci | ³²P dCTP |
| 7 units | DNA polymerase I, Klenow fragment. |

It was found that the addition of polyethylene glycol 6000 to a final concentration of 4% increased the incorporation of radioactivity up to three fold.

The reaction mix was incubated for 2 hrs at 20°C.

At the end of the incubation, unincorporated nucleotides were removed by washing the SP-cellulose four times by centrifugation. The washing buffer was 140mM sodium chloride, 15mM sodium citrate and 20mM sodium phosphate, pH 7.0. The sample was vortexed for 1 minute at room temperature then centrifuged at 12,000g for 5 minutes. This was repeated four times.

Finally, the SP-cellulose was added to a vial containing scintillation solution and incorporation was determined using a liquid scintillation counter.

### Example 2

In this example of the detection of a specific polynucleotide sequence, single stranded genomic DNA from the bacteriophage M13 mp18 was detected using the following oligonucleotide primer:

M13 ssDNA
3' . . . T T T G C T G C C G G T C A C G G T T C G A A . . . 5'
5' A A A C G A C G G C C A G T G C C
Specific Primer

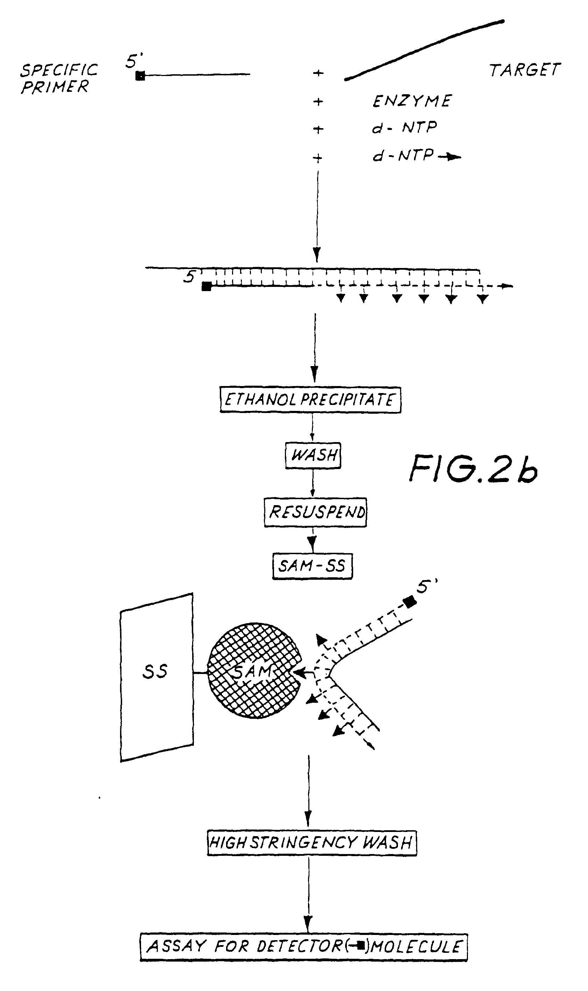

The configuration used in this example is demonstrated in Figure 2b; the detector molecule in this example is ³²P and is attached to the 5' end of the specific primer; the capture molecule is biotin and is incorporated into the extended primer as biotin-16-dUTP (an analogue of dTTP); the specific affinity molecule is streptavidin and the solid support is agarose.

The specific primer was synthesised using an oligonucleotide synthesiser and was 5' end labelled with ³²P using polynucleotide kinase as follows:

| Reaction mix: | |
|---|---|
| Specific primer (50 pmoles 5'OH ends) | 1µl |
| 100 mM dithiothreitol | 5µl |
| 10x TM Buffer (600 mM Tris, pH7.6; 90 mM MgCl₂) | 5µl |
| γ-³²P-dATP (3000 Ci/mmole) | 15µl |
| T₄ polynucleotide kinase (8.6 units/ml) | 2µl |
| ddH₂O | 22µl |

The reaction mix was incubated at 37°C for 20 minutes. The labelled primer was separated from unincorporated ³²P using a DEAE cellulose column.

The primer extension reaction was carried using the following reaction mix either with M13 mp18 or lambda bacteriophage (as a non-homologous control):

| | M13 | Lambda |
|---|---|---|
| Template DNA (500 ng) | 2.5ul | 10ul |
| ³²P labelled specific primer | 10ul | 10ul |
| DNA polymerase (Klenow; 1 unit/ul | 1ul | 1ul |
| Biotin-16-dUTP (1 mM) | 1ul | 1ul |
| dCTP (1 mM) | 1ul | 1ul |
| dGTP (1 mM) | 1ul | 1ul |
| dATP (1 mM) | 1ul | 1ul |
| NaCl (5 M) | 1ul | 1ul |
| MgSO₄ (1 M) | 0.5ul | 0.5ul |
| Dithiothreitol (4 mM) | 1.25ul | 1.25ul |
| Bovine serum albumin (1 mg/ml) | 2.5ul | 2.5ul |
| Tris HCl, pH7.0 (1 M) | 2.5ul | 2.5ul |
| ddH₂O | 24.75ul | 25/25ul |

The reaction mix was incubated at 37°C for 60 minutes. At the completion of the incubation, the extended primer was precipitated with ethanol to remove unincorporated biotin-16-dUTP. To the reaction mix was added one volume (50ul) 5M ammonium acetate and 250ul ethanol, followed by brief mixing and incubation at -20°C for one hour. The precipitate was collected by centrifugation, dried briefly, resuspended in 50ul ddH₂O and applied to a 200ul strepta-vidin/agarose column (containing 0.12mg streptavidin) previously equilibrated with binding buffer (10 mM Tris HCl, pH7.5; 200 mM NaCl; 1 mM EDTA). The column was washed five times with 500ul each of binding buffer. The streptavidin/agarose was suspended in Iml binding buffer to which was added 6ml of scintillation fluid and the mixture counted in a liquid scintillation counter.

The results for the M13 and the lambda systems were as follows:

| Template | cpm |
|---|---|
| M13 | 29,055 |
| lambda | 1,043 |

The results demonstrate that the primer was specifically extended only in the presence of the homologous template, M13, but not extended in the presence of the non-homologous template, lambda. Further, biotin was incorporated into the extended primer.

### Example 3

In this example of the detection of an altered nucleotide base, the configuration shown in Figure 3c was used. The M13 sequence detected and the oligonucleotide primer sequence used were as follows:

M13 ssDNA
3' . . . T T T G C T G C C G G T C A C G G T T C G A A . . . 5'
5' A A A C G A C G G C C A G T G C C
Specific Primer

The specific primer was synthesised using an oligonucleotide synthesiser.

The reaction mix was set up as follows:

| | |
|---|---|
| 500 ng M13mp18 ssDNA | 2.5µl |
| 0.8 pmoles specific primer | 2.0µl |
| 10x reaction buffer | 1.5µl |
| ddH₂O | 1.0µl |

This reaction mix was incubated at 55°C for 10 minutes at which time the following were added:

| | |
|---|---|
| ³²P dATP (3000 Ci/mmole, 10 mCi/ml) | 0.5µl |
| ³²P ddATP (3000 Ci/mmole, 10 mCi/ml) | 1.5µl |
| DNA polymerase (Klenow) (1 unit/µl) | 1.0µl |

This reaction mix was incubated for a further 15 minutes at 25°C. The reaction was stopped by the addition of 4ul formamide dye (95% formamide, 0.1% xylene cyanol, 0.1% bromophenol blue). The reaction mix was heated at 100°C for three minutes prior to loading on a 20% polyacrylamide gel and electrophoresing for 30 minutes at 30 milliamps. The gel was then fixed in 10% acetic acid, washed with ddH₂O and exposed to X-ray film at -80°C for 12 hou rs.

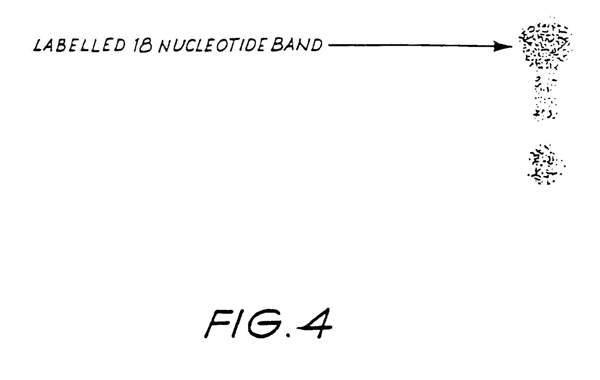

The resulting autoradiograph (Figure 4) exhibited only one band of 18 nucleotides in length. Thus the primer had been extended by only one nucleotide by the incorporation of a labelled adenine residue and thus detecting a specific single nucleotide in a nucleic acid sequence.

### Example 4

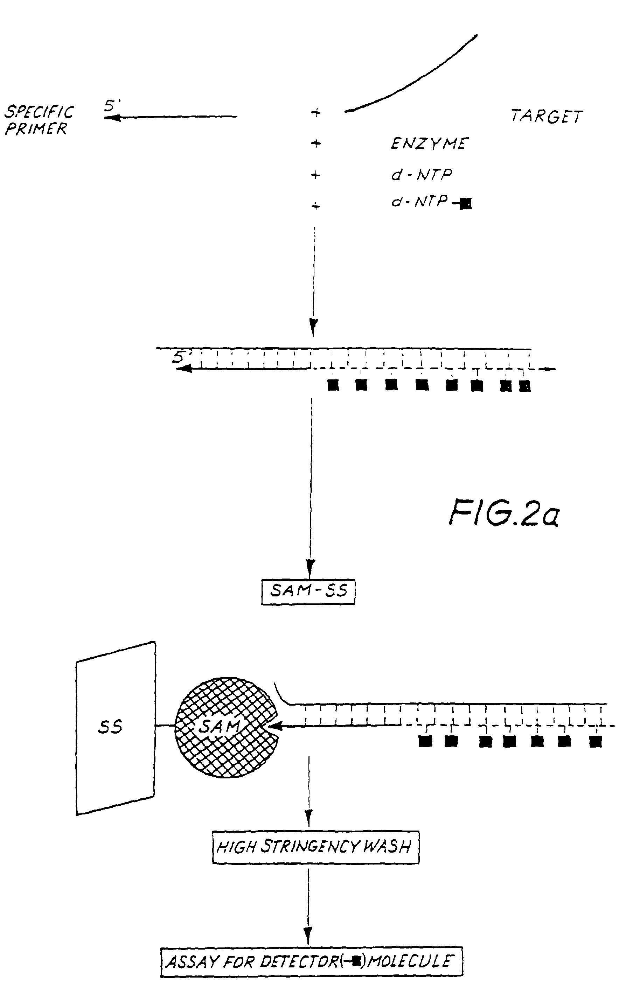

In this example of the detection of a specific polynucleotide sequence, the method of Fig. 2a was used for detection of the single stranded genomic DNA of the bacteriophage M13.

### 1. Synthesis of the synthetic primer

A 25-mer DNA complementary to MIS ssDNA was synthesized by Clontec (USA).

The 25-mer had the nucleotide sequence 5'-X ACG TTG TAA AAC GAC GGC CAG TGC C-3' where X is biotin covalently attached to a modified nucleotide.

### 2. Hybridisation and primer extension

The primer was mixed with buffer containing either M13 DNA (target) or herring sperm DNA (control) and extended using DNA Polymerase I (Klenow fragment).

Reaction conditions were as follows:
50 mM Tris pH 7.5
10 mM MgCl₂
4 mM DTT
100 mM NaCl
50 ug/ml bovine serum albumin
0.02 mM each dATP, dGTP, dTTP
1 Ci ³²P dCTP
742.5ng/50ul M13 or herring sperm DNA
25.8ng/50ul biotin-primer
0.5 units DNA polymerase I, Klenow fragment.

The reaction mixes were incubated at 37°C for 60' and terminated by precipitation and washing.

Precipitation was carried out by addition of 5M ammonium acetate, calf thymus DNA as carrier and ethanol. Pellets collected by centrifugation were washed with 70% ethanol until washings contained less than 500 cpm.

Washed pellets were then dissolved in binding buffer consisting of 200mM NaCl, 10mH Tris pH 7.5, 1 mH EDTA and applied to a column of streptavidin-agarose. Columns were washed with several volumes of buffer and counts bound to streptavidin agarose (SA) were measured, with the following results:

| | |
|---|---|
| Target | cpm bound to SA |
| M13 | 11,939 |
| Herring Sperm | 83 |

The results demonstrate that only primers extended in the presence of target M13 DNA result in significant radioactivity being bound to the column.

### Industrial Applicability

The method of the invention can be readily used in the following applications:
a) the simple, rapid, sensitive and automatable detection of infectious diseases of humans, animals and plants;
b) the specific, rapid and large scale detection of base changes in nucleic acid sequences, particularly in the detection of gene defects and DNA fingerprinting;
c) kits for use with the above applications; and
d) automated equipment for use with the above applications.

## Claims

1. A method for detecting whether a specific nucleotide or base is at a particular position in a specific polynucleotide sequence in material comprising:
a) exposing, under hybridizing conditions, said material to an oligonucleotide primer having a sequence complementary to part of the specific polynucleotide sequence wherein said primer incorporates a separation element or a detectable element at the 5' end of said primer and wherein said primer binds to part of said specific polynucleotide sequence in said material (i) immediately adjacent to the particular position or (ii) not immediately adjacent to the particular position whereby there is an intervening sequence between the particular position and primer bound to the specific polynucleotide sequence;
b) extending primer bound to the specific polynucleotide sequence with the proviso that the bound primer is only extended up to and including said specific nucleotide or base when said specific nucleotide or base is at the particular position in the specific polynucleotide sequence wherein the extended primer has a detectable element and/or a separation element at a position complementary to said specific nucleotide or base with the proviso that said extended primer has at least one separation element and at least one detectable element and, when there is an intervening sequence between the particular position and primer bound to the specific polynucleotide sequence, with the further proviso that the intervening sequence cannot be one where bases or nucleotides complementary to the intervening sequence and which are incorporated into the extended primer cause incorporation of an interfering detectable and/or separation element;
c) separating any extended primer into a fraction wherein said fraction does not have detectable element not included in said extended primer with the proviso that said separating does not include the step of digesting the specific polynucleotide sequence having said extended primer bound thereto; and
d) determining whether said extended primer is present in said fraction by assaying said fraction for said extended primer wherein the presence of said extended primer indicates that the specific nucleotide or base is at the particular position in the specific polynucleotide sequence and the absence of said extended primer indicates that the specific nucleotide or base is not at the particular position in the specific polynucleotide sequence;
wherein said extending comprises using at least one dideoxynucleotide, said dideoxynucleotide being complementary to said specific nucleotide or base.

2. A method for detecting whether the same or different specific nucleotides or bases are at particular positions in at least two different specific polynucleotide sequences in material having a plurality of different polynucleotide sequences comprising:
a) exposing, under hybridizing conditions, said material to at least two different oligonucleotide primers each of said different oligonucleotide primers having a sequence complementary to part of one of said different specific polynucleotide sequences wherein:
each of said primers binds to its complementary polynucleotide sequence when present in said material,
each of said oligonucleotide primers binding to part of a different specific polynucleotide sequence to that of the other primer(s),
each of said primers binds to its complementary specific polynucleotide sequence in said material (i) immediately adjacent to the particular position or (ii) not immediately adjacent to the particular position whereby there is an intervening sequence between the particular position and primer bound to the specific polynucleotide sequence when present in said material, and
each of said primers incorporates a separation element or a detectable element at the 5' end of said primer;
b) extending said different oligonucleotide primers bound to their complementary polynucleotide sequences with the proviso that each of the bound primers is only extended up to and including the particular position when said specific nucleotide or base is at the particular position in the specific nucleotide sequence wherein each of said extended primers has a detectable element and/or a separation element at a position complementary to said specific nucleotide or base, with the further proviso that each of said extended primers has at least one separation element and at least one detectable element and, when there are intervening sequences between the particular positions and primers bound to the specific polynucleotide sequences, with the further proviso that the intervening sequences cannot be ones where bases or nucleotides complementary to the intervening sequences and which are incorporated into the extended primer(s) cause incorporation of interfering detectable and/or separation element(s);
c) separating any extended primer(s) which has extended only up to a specific nucleotide or base at a particular position into at least one fraction wherein said fraction does not have detectable element not included in said extended primer with the proviso said separating does not include the step of digesting the specific polynucleotide sequence having said extended primer bound thereto; and
d) determining whether any of said extended primers are present in said fraction(s) by assaying said fraction (s) for said extended primer(s) wherein the presence of an extended primer indicates that a specific nucleotide or base is at a particular position in a specific polynucleotide sequence and the absence of said extended primer(s) in a fraction(s) indicates that at least one specific nucleotide or base is not at a particular position in at least one different specific polynucleotide sequence;
wherein said extending comprises using at least one dideoxynucleotide, said dideoxynucleotide being complementary to said specific nucleotide or base.

3. A screening method for detecting whether the same or different specific nucleotides or bases are at particular positions in at least two different specific polynucleotide sequences in material having a plurality of different polynucleotide sequences comprising:
a) exposing, under hybridizing conditions, said material to at least two different oligonucleotide primers each
of said different oligonucleotide primers having a sequence complementary to part of one of said different specific polynucleotide sequences wherein:
each of said primers binds to its complementary polynucleotide sequence when present in said material
each of said oligonucleotide primers binding to part of a different specific polynucleotide sequence to that of the other primer(s),
each of said primers binds to its complementary specific polynucleotide sequence in said material (i) immediately adjacent to the particular position or (ii) not immediately adjacent to the particular position whereby there is an intervening sequence between the particular position and primer bound to the specific polynucleotide sequence when present in said material, and
each of said primers incorporates a separation element or a detectable element at the 5' end of said primer;
b) extending said different oligonucleotide primers bound to their complementary polynucleotide sequences with the proviso that each of the bound primers is only extended up to and including the particular position when said specific nucleotide or base is at the particular position in the specific nucleotide sequence wherein each of said extended primers has a detectable element and/or a separation element at a position complementary to said specific nucleotide or base, with the further proviso that each of said extended primers has at least one separation element and at least one detectable element and, when there are intervening sequences between the particular positions and primers bound to the specific polynucleotide sequences, with the further proviso that the intervening sequences cannot be ones where bases or nucleotides complementary to the intervening sequences and which are incorporated into the extended primer(s) cause incorporation of interfering detectable and/or separation element(s);
c) separating any extended primer(s) which has extended only up to a specific nucleotide or base at a particular position into a fraction wherein said fraction does not have detectable element not included in said extended primer with the proviso said separating does not include the step of digesting the specific polynucleotide sequences having said extended primers bound thereto; and
d) determining whether at least one of said extended primers is present in said fraction by assaying said fraction for all of said extended primers wherein the presence of an extended primer indicates that at least one specific nucleotide or base is at a particular position in a specific polynucleotide sequence and the absence of all said extended primers in said fraction indicates that at least one specific nucleotide or base is not at a particular position in any of the different specific polynucleotide sequences;
wherein said extending comprises using at least one dideoxynucleotide, said dideoxynucleotide being complementary to said specific nucleotide or base.

4. The method of any one of claims 1 to 3 wherein said primer binds to part of said specific polynucleotide sequence in said material immediately adjacent to the particular position.

5. The method of any one of claims 1 to 3 wherein said primer binds to part of said specific polynucleotide sequence in said material not immediately adjacent to the particular position whereby there is an intervening sequence between the particular position and primer bound to the specific polynucleotide sequence.

6. The method of claim 2 or claim 3 wherein each of said primers incorporates a different separation element.

7. The method of any one of claims 1 to 6 wherein said extending comprises using four different nucleotides selected from the group consisting of dATP, dCTP, dGTP, dTTP, dUTP, dITP, ddATP, ddCTP, ddGTP, ddITP and ddTTP, at least one of said nucleotides being selected from the group consisting of ddATP, ddCTP, ddGTP, ddITP and ddTTP, said nucleotides incorporating a detectable element and/or a separation element.

8. The method of any one of claims 1 to 6 wherein said extending comprises using one nucleotide selected from the group consisting of ddITP, ddATP, ddCTP, ddGTP, and ddTTP, said nucleotide incorporating a detectable element and/or a separation element.

9. The method of claim 7 or claim 8 wherein at least one of said nucleotides includes a detectable element.

10. The method of claim 7 or claim 8 wherein at least one of said nucleotides includes a separation element.

11. The method of claim 7 or claim 8 wherein at least one of said nucleotides includes separation and detectable elements.

12. The method of any one of claims 1 to 8 wherein said separating step comprises: contacting any extended primer with a molecule having affinity for said separation element; and separating said contacted extended primer(s) to provide said fraction wherein said separation element and said molecule are selected from the group listed as follows:
| **Separation Element** | | **Molecule Having Affinity for Separation Element** |
|---|---|---|
| (a) | a ligand for which there is a receptor | a receptor for the ligand; |
| (b) | a receptor; | the ligand for the receptor; |
| (c) | an antigen; | an antibody for the antigen; |
| (d) | an antibody for an antigen | the antigen; |
| (e) | an antiidiotypic antibody | an antibody for the antiidiotypic antibody; |
| (f) | an antibody for an antiidiotypic antibody; | an antiidiotypic antibody for the antibody |
| (g) | a haptenic group; | an antibody for the haptenic group; |
| (h) | an antibody for a haptenic group; | the haptenic group |
| (i) | an enzyme; | a binding inhibitor for the enzyme; and |
| (j) | a binding inhibitor for an enzyme; | the enzyme. |

13. The method of claim 1 wherein said separating step comprises: contacting any extended primer with a molecule having affinity for the separation element, said molecule being linked to a support that facilitates said separating; and separating said contacted extended primer to provide said fraction.

14. The method of claim 2 or claim 3 wherein said separating step comprises: contacting any extended primers with molecules having affinity for the separation elements, said molecules being linked to supports that facilitate said separating; and separating said contacted extended primers to provide said fraction.

15. The method of claims 13 or 14 wherein said separation element(s) and said molecule(s) are selected from the group listed as follows:
| **Separation Element** | | **Molecule Having Affinity for Separation Element** |
|---|---|---|
| (a) | a ligand for which there is a specific receptor | a specific receptor for the ligand |
| (b) | a specific receptor; | the ligand for the specific receptor; |
| (c) | an antigen; | a specific antibody for the antigen; |
| (d) | a specific antibody for an antigen; | the antigen; |
| (e) | an antiidiotypic antibody | a specific antibody for the antiidiotypic antibody; |
| (f) | an antibody for an antiidiotypic antibody; | an antiidiotypic antibody specific for the antibody |
| (g) | a haptenic group; | a specific antibody for the haptenic group; |
| (h) | a specific antibody for a haptenic group; | the haptenic group |
| (i) | an enzyme; | a tight binding inhibitor for the enzyme; and |
| (j) | a tight binding inhibitor for an enzyme; | the enzyme. |

16. The method of claim 13 wherein said support is a test strip.

17. The method of claim 14 or claim 15 wherein said supports are test strips.

18. The method of any one of claims 1 to 17 wherein the specific polynucleotide sequence(s) is a DNA sequence.

19. The method of any one of claims 1 to 17 wherein the specific polynucleotide sequence(s) is an RNA sequence.

20. The method of any one of claims 1 to 17 wherein the specific polynucleotide sequence(s) is from an infectious or disease causing organism.

21. The method of any one of claims 1 to 8 wherein the oligonucleotide primer incorporates a detectable element at its 5' end.

22. The method of claim 21 wherein said separating comprises precipitating any extended primer(s) and resuspending said extended primer(s) in a solution conducive to binding of said separation element(s) to a molecule(s) having affinity for said separation element(s).

23. The method of claim 1 wherein step d) comprises:
d) determining whether said specific polynucleotide sequence having said extended primer bound thereto is present in said fraction by assaying said fraction for said extended primer wherein the presence of said extended primer indicates that the specific nucleotide or base is at the particular position in the specific polynucleotide sequence and the absence of said extended primer indicates that the specific nucleotide or base is not at the particular position in the specific polynucleotide sequence.

24. The method of claim 2 wherein step d) comprises:
d) determining whether any of the specific polynucleotide sequences having said extended primers bound thereto are present in said fraction(s) by assaying said fraction(s) for said extended primer(s) wherein the presence of an extended primer indicates that a specific nucleotide or base is at a particular position in a specific polynucleotide sequence and the absence of said extended primer(s) in a fraction(s) indicates that at least one specific nucleotide or base is not at a particular position in at least one different specific polynucleotide sequence.

25. The method of claim 3 wherein step d) comprises:
d) determining whether at least one specific polynucleotide sequence having one of said extended primers bound thereto is present in said fraction by assaying said fraction for all of said extended primers wherein the present of an extended primer indicates that at least one specific nucleotide or base is at a particular position in a specific polynucleotide sequence and the absence of all said extended primers in said fraction indicates that at least one specific nucleotide or base is not at a particular position in any of the different specific polynucleotide sequences.

## Patentansprüche

1. Verfahren zur Bestimmung, ob sich ein spezielles Nucleotid oder eine spezielle Base an einer bestimmten Position in einer speziellen Polynucleotid-Sequenz in einem Material befindet, aufweisend:
a) Aussetzen des Materials, unter hybridisierenden Bedingungen, einem Oligonucleotid-Primer mit einer zu einem Teil der speziellen Polynucleotid-Sequenz komplementären Sequenz, wobei der Primer an seinem 5'-Ende ein Trennelement oder ein nachweisbares Element beinhaltet, und wobei der Primer an einen Teil der speziellen Polynucleotid-Sequenz in dem Material (i) direkt angrenzend an die bestimmte Position bindet oder (ii) nicht direkt angrenzend an die bestimmte Position bindet, wodurch es eine intervenierende Sequenz zwischen der bestimmten Position und an die spezielle Polynucleotid-Sequenz gebundenem Primer gibt;
b) Verlängern von an die spezielle Polynucleotid-Sequenz gebundenem Primer mit der Maßgabe, daß der gebundene Primer nur bis zu und einschließlich des speziellen Nucleotids oder der speziellen Base verlängert wird, wenn sich das spezielle Nucleotid oder die spezielle Base an der bestimmten Position in der speziellen Polynucleotid-Sequenz befindet, wobei der verlängerte Primer ein nachweisbares Element und/oder ein Trenn-Element an einer zu dem speziellen Nucleotid- oder der speziellen Base komplementären Position besitzt, mit der Maßgabe, daß der verlängerte Primer mindestens ein Trenn-Element und mindestens ein nachweisbares Element besitzt und, wenn es eine intervenierende Sequenz zwischen der bestimmten Position und an die spezielle Polynucleotid-Sequenz gebundenem Primer gibt, mit der weiteren Maßgabe, daß die intervenierende Sequenz keine sein kann, bei der Basen oder Nucleotide, die zu der intervenierenden Sequenz komplementär sind und die in den verlängerten Primer inkorporiert werden, ein Inkorporieren eines störenden nachweisbaren und/oder Trenn-Elements verursachen;
c) Abtrennen von irgendwelchem verlängerten Primer in eine Fraktion, wobei die Fraktion kein nicht in dem verlängerten Primer enthaltenes nachweisbares Element besitzt, mit der Maßgabe, daß das Abtrennen nicht den Schritt des Verdaus der speziellen Polynucleotid-Sequenz mit dem daran gebundenen verlängerten Primer beinhaltet; und
d) Bestimmen, ob der verlängerte Primer in der Fraktion anwesend ist, durch Untersuchen der Fraktion auf den verlängerten Primer, wobei die Anwesenheit des verlängerten Primers anzeigt, daß sich das spezielle Nucleotid oder die spezielle Base an der bestimmten Position in der speziellen Polynucleotid-Sequenz befindet, und das Fehlen des verlängerten Primers anzeigt, daß sich das spezielle Nucleotid oder die spezielle Base nicht an der bestimmten Position in der speziellen Polynucleotid-Sequenz befindet;
bei dem das Verlängern die Verwendung von mindestens einem Didesoxynucleotid umfaßt, wobei das Didesoxynucleotid zu dem speziellen Nucleotid oder der speziellen Base komplementär ist.

2. Verfahren zur Bestimmung, ob sich an bestimmten Positionen in mindestens zwei verschiedenen speziellen Polynucleotid-Sequenzen in einem Material mit einer Mehrzahl verschiedener Polynucleotid-Sequenzen dieselben oder verschiedene spezielle Nucleotide oder Basen befinden, aufweisend:
a) Aussetzen, unter hybridisierenden Bedingungen, des Materials mindestens zwei verschiedenen Oligonucleotid-Primern, wobei jeder der verschiedenen Oligonucleotid-Primer eine zu einem Teil einer der verschiedenen Polynucleotid-Sequenzen komplementäre Sequenz hat, wobei;
jeder der Primer an seine komplementäre Polynucleotid-Sequenz bindet, wenn sie in dem Material vorhanden ist, wobei jeder der Oligonucleotid-Primer an einen Teil einer von derjenigen des (der) anderen Primer (s) verschiedenen speziellen Polynucleotid-Sequenz bindet,
jeder der Primer an seine komplementäre spezielle Polynucleotid-Sequenz in dem Material (i) direkt angrenzend an die bestimmte Position bindet oder (ii) nicht direkt angrenzend an die bestimmte Position bindet, wodurch es eine intervenierende Sequenz zwischen der bestimmten Position und an die spezielle Polynucleotid-Sequenz, wenn sie in dem Material vorhanden ist, gebundenem Primer gibt, und
jeder der Primer ein Trenn-Element oder ein nachweisbares Element an seinem 5'-Ende beinhaltet;
b) Verlängern der verschiedenen, an ihre komplementären Polynucleotid-Sequenzen gebundenen Oligonucleotid-Primer mit der Maßgabe, daß jeder der gebundenen Primer nur bis zu und einschließlich der bestimmten Position verlängert wird, wenn sich das spezielle Nucleotid oder die spezielle Base an der bestimmten Position in der speziellen Nucleotid-Sequenz befindet, wobei jeder der verlängerten Primer ein nachweisbares Element und/oder ein Trennelement an einer zu dem speziellen Nucleotid oder der speziellen Base komplementären Position besitzt, mit der weiteren Maßgabe, daß jeder der verlängerten Primer mindestens ein Trenn-Element und mindestens ein nachweisbares Element besitzt und, wenn es intervenierende Sequenzen zwischen den bestimmten Positionen und an die speziellen Polynucleotid-Sequenzen gebundenen Primem gibt, mit der weiteren Maßgabe, daß die intervenierenden Sequenzen keine sein können, bei denen Basen oder Nucleotide, die zu den intervenierenden Sequenzen komplementär sind und die in den (die) verlängerten Primer inkorporiert werden, ein Inkörporieren eines störenden nachweisbaren und/oder Trenn-Elements oder von störenden nachweisbaren und/oder Trenn-Elementen verursachen;
c) Abtrennen von irgendwelchem (irgendwelchen) verlängerten Primer (n), der (die) nur bis zu einem speziellen Nucleotid oder einer speziellen Base in einer bestimmten Position verlängert wurde (n), in mindestens eine Fraktion, wobei die Fraktion kein nicht in dem verlängerten Primer enthaltenes nachweisbares Element besitzt, mit der Maßgabe, daß das Abtrennen nicht den Schritt des Verdaus der speziellen Polynucleotid-Sequenz mit dem daran gebundenen Primer enthält; und
d) Bestimmen, ob irgendwelche der verlängerten Primer in der (den) Fraktion(en) anwesend sind, durch Untersuchen der Fraktion(en) auf den (die) verlängerten Primer, wobei die Anwesenheit eines verlängerten Primers anzeigt, daß sich ein spezielles Nucleotid oder eine spezielle Base an einer bestimmten Position in einer speziellen Polynucleotid-Sequenz befindet, und das Fehlen des (der) verlängerten Primer(s) in der (den) Fraktion(en) anzeigt, daß sich mindestens ein spezielles Nucleotid oder eine spezielle Base nicht an einer bestimmten Position in mindestens einer der verschiedenen speziellen Polynucleotid-Sequenzen befindet;
wobei das Verlängern die Verwendung von mindestens einem Didesoxynucleotid umfaßt, wobei das Didesoxynucleotid zu dem speziellen Nucleotid oder der speziellen Base komplementär ist.

3. Screening-Verfahren zur Bestimmung, ob sich dieselben oder verschiedene spezielle Nucleotide oder Basen an bestimmten Positionen in mindesiens zwei verschiedenen speziellen Polynucleotid-Sequenzen in einem Material mit einer Mehrzahl verschiedener Polynucleotid-Sequenzen befinden, aufweisend:
a) Aussetzen des Materials, unter hybridisierenden Bedingungen, mindestens zwei verschiedenen Oligonucleotid-Primern, wobei jeder der verschiedenen Oligonucleotid-Primer eine zu einem Teil einer der verschiedenen speziellen Polynucleotid-Sequenzen komplementäre Sequenz hat, wobei:
jeder der Primer an seine komplementäre Polynucleotid-Sequenz bindet, wenn sie in dem Material vorhanden ist, wobei jeder der Oligonucleotid-Primer an einen Teil einer speziellen Polynucleotid-Sequenz, die von der des (der) anderen Primer(s) verschieden ist, bindet,
jeder der Primer an seine komplementäre spezielle Polynucleotid-Sequenz in dem Material (i) direkt angrenzend an die bestimmte Position bindet oder (ii) nicht direkt angrenzend an die bestimmte Position bindet, wodurch es eine intervenierende Sequenz zwischen der bestimmten Position und an die spezielle Polynucleotid-Sequenz, wenn sie in dem Material vorhanden ist, gebundenem Primer gibt, und
jeder der Primer ein Trenn-Element oder ein nachweisbares Element an seinem 5'-Ende beinhaltet;
b) Verlängern der verschiedenen, an ihre komplementären Polynucleotid-Sequenzen gebundenen Oligonucleotid-Primer mit der Maßgabe, daß jeder der gebundenen Primer nur bis zu und einschließlich der bestimmten Position verlängert wird, wenn sich das spezielle Nucleotid oder die spezielle Base an der bestimmten Position in der speziellen Nucleotid-Sequenz befindet, wobei jeder der verlängerten Primer ein nachweisbares Element und/oder ein Trenn-Element an einer zu dem speziellen Nucleotid oder der speziellen Base komplementären Position besitzt, mit der weiteren Maßgabe, daß jeder der verlängerten Primer mindestens ein Trenn-Element und mindestens ein nachweisbares Element besitzt und, wenn es intervenierende Sequenzen zwischen den bestimmten Positionen und an die speziellen Polynucleotid-Sequenzen gebundenen Primem gibt, mit der weiteren Maßgabe, daß die intervenierenden Sequenzen keine sein können, bei denen Basen oder Nucleotide, die zu den intervenierenden Sequenzen komplementär sind und die in den (die) verlängerten Primer inkorporiert werden, ein Inkorporieren eines störenden nachweisbaren und/oder Trenn-Elements oder störender nachweisbarer und/oder Trenn-Elemente verursachen;
c) Abtrennen von irgendwelchem (irgendwelchen) verlängerten Primer(n), der (die) nur bis zu einem speziellen Nucleotid oder bis zu einer speziellen Base an einer bestimmten Position verlängert wurde(n), in eine Fraktion, wobei die Fraktion kein nicht in dem verlängerten Primer enthaltenes nachweisbares Element besitzt, mit der Maßgabe, daß das Abtrennen nicht den Schritt des Verdaus der speziellen Polynucleotid-Sequenzen mit den daran gebundenen verlängerten Primem beinhaltet; und
d) Bestimmen, ob mindestens einer der verlängerten Primer in der Fraktion vorhanden ist, durch Untersuchen der Fraktion auf alle der verlängerten Primer, wobei die Anwesenheit eines verlängerten Primers anzeigt, daß sich mindestens ein spezielles Nucleotid oder eine spezielle Base an einer bestimmten Position in einer speziellen Polynucleotid-Sequenz befindet, und das Fehlen aller der verlängerten Primer in der Fraktion anzeigt, daß sich mindestens ein spezielles Nucleotid oder mindestens eine spezielle Base nicht in einer bestimmten Position in irgendeiner der verschiedenen speziellen Polynucleotid-Sequenzen befindet;
bei dem das Verlängern die Verwendung von mindestens einem Didesoxynucleotid umfaßt, wobei das Didesoxynucleotid zu dem speziellen Nucleotid oder der speziellen Base komplementär ist.

4. Verfahren nach einem der Ansprüche 1 bis 3, bei dem der Primer an einen direkt an die bestimmte Position angrenzenden Teil der speziellen Polynucleotid-Sequenz in dem Material bindet.

5. Verfahren nach einem der Ansprüche 1 bis 3, bei dem der Primer an einen nicht direkt an die bestimmte Position angrenzenden Teil der speziellen Polynucleotid-Sequenz in dem Material bindet, wodurch es eine intervenierende Sequenz zwischen der bestimmten Position und an die spezielle Polynucleotid-Sequenz gebundenem Primer gibt.

6. Verfahren nach Anspruch 2 öder 3, bei dem jeder der Primer ein verschiedenes Trenn-Element beinhaltet.

7. Verfahren nach einem der Ansprüche 1 bis 6, bei dem das Verlängern die Verwendung von vier verschiedenen Nucleotiden umfaßt, die ausgewählt sind aus der Gruppe, die besteht aus dATP, dCTP, dGTP, dTTP, dUTP, dITP, ddATP, ddCTP, ddGTP, ddITP und ddTTP, wobei mindestens eines der Nucleotide ausgewählt ist aus der Gruppe, die besteht aus ddATP, ddCTP, ddGTP, ddITP und ddTTP, wobei die Nucleotide ein nachweisbares Element und/oder ein Trenn-Element beinhalten.

8. Verfahren nach einem der Ansprüche 1 bis 6, bei dem das Verlängern die Verwendung eines Nucleotids umfaßt, das ausgewählt ist aus der Gruppe, die besteht aus ddITP, ddATP, ddCTP, ddGTP und ddTTP, wobei das Nucleotid ein nachweisbares Element und/oder ein Trenn-Element beinhaltet.

9. Verfahren nach Anspruch 7 oder 8, bei dem mindestens eines der Nucleotide ein nachweisbares Element enthält.

10. Verfahren nach Anspruch 7 oder 8, bei dem mindestens eines der Nucleotide ein Trenn-Element enthält.

11. Verfahren nach Anspruch 7 oder 8, bei dem mindestens eines der Nucleotide Trenn- und nachweisbare Elemente enthält

12. Verfahren nach einem der Ansprüche 1 bis 8, bei dem der Trenn-Schritt aufweist: in Berührung bringen von irgendwelchem verlängerten Primer mit einem Molekül, das Affinität zu dem Trenn-Element besitzt; und Abtrennen des (der) in Berührung gebrachten verlängerten Primer(s), um die Fraktion zu liefern, in der das Trenn-Element und das Molekül ausgewählt sind aus der wie folgt aufgelisteten Gruppe:
| **Trenn-Element** | | **Molekül mit Affinltät für das Trenn-Element** |
|---|---|---|
| a) | ein Ligand, für den es einen Rezeptor gibt | einen Rezeptor für den Liganden; |
| | | |
| b) | ein Rezeptor | der Ligand für den Rezeptor |
| | | |
| c) | ein Antigen | ein Antikörper für das Antigen |
| | | |
| d) | ein Antikörper für ein Antigen | das Antigen; |
| | | |
| e) | ein anti-idiotyper Antikörper | ein Antikörper für den anti-idiotypen Antikörper; |
| | | |
| f) | ein Antikörper für einen anti-idiotypen Antikörper | ein anti-idiotyper Antikörper für den Antikörper; |
| | | |
| g) | eine Hapten-Gruppe | ein Antikörper für die Hapten-Gruppe; |
| | | |
| h) | ein Antikörper für eine Hapten-Gruppe | die Hapten-Gruppe; |
| | | |
| i) | ein Enzym | ein Bindungs-Hammer für das Enzym; und |
| | | |
| j) | ein Bindungs-Hemmer für ein Enzym | das Enzym. |

13. Verfahren nach Anspruch 1, bei dem der Trenn-Schritt aufweist: in Berührung bringen von irgendwelchem verlängerten Primer mit einem Molekül, das Affinität zu dem Trenn-Element besitzt, wobei das Molekül an einen Träger, der die Trennung erleichtert, gebunden ist; und Abtrennen des in Berührung gebrachten verlängerten Primers, um die Fraktion zu liefern.

14. Verfahren nach Anspruch 2 oder 3, bei dem der Trenn-Schritt aufweist; in Berührung bringen von irgendwelchen verlängerten Primem mit Molekülen, die Affinität für die Trenn-Elemente besitzten, wobei die Moleküle an Träger, die das Trennen erleichtern, gebunden sind; und Abtrennen der in Berührung gebrachten verlängerten Primer, um die Fraktion zu liefern.

15. Verfahren nach Anspruch 13 oder 14, bei dem das (die) Trenn-Element(e) und das (die) Molekül(e) ausgewählt ist (sind) aus der wie folgt aufgelisteten Gruppe:
| **Trenn-Element** | | **Molekül mit Affinität für das Trenn-Element** |
|---|---|---|
| a) | ein Ligand, für den es einen spezifischen Rezeptor gibt | ein spezifischer Rezeptor für den Liganden; |
| | | |
| b) | ein spezifischer Rezeptor | der Ligand für den spezifischen Rezeptor; |
| | | |
| c) | ein Antigen | ein spezifischer Antikörper für das Antigen; |
| | | |
| d) | ein spezifischer Antikörper für ein Antigen | das Antigen; |
| | | |
| e) | ein anti-idiotyper Antikörper | ein spezifischer Antikörper für den anti-idiotypen Antikörper; |
| | | |
| f) | ein Antikörper für einen anti-idiotypen Antikörper | ein für den Antikörper spezifischer anti-idiotyper Antikörper; |
| | | |
| g) | eine Hapten-Gruppe | ein spezifischer Antikörper für die Hapten-Gruppe; |
| | | |
| h) | ein spezifischer Antikörper für eine Hapten-Gruppe | die Hapten-Gruppe; |
| | | |
| i) | ein Enzym | ein festsitzender Bindungs-Inhibitor für das Enzym; und |
| | | |
| j) | ein festsitzender Bindungs-Inhibitor für ein Enzym | das Enzym. |

16. Verfahren nach Anspruch 13, bei dem der Träger ein Prüfstreifen ist.

17. Verfahren nach Anspruch 14 oder 15, bei dem die Träger Prüfstreifen sind.

18. Verfahren nach einem der Ansprüche 1 bis 17, bei dem die spezielle(n) Polynucleotid-Sequenz(en) (eine) DNA-Sequenz(en) ist (sind).

19. Verfahren nach einem der Ansprüche 1 bis 17, bei dem die spezielle(n) Polynucleotid-Sequenz(en) (eine) RNA-Sequenz(en) ist (sind).

20. Verfahren nach einem der Ansprüche 1 bis 17, bei dem die spezielle(n) Polynucleotid-Sequenz(en) von einem infektiösen oder Krankheit verursachenden Organismus stammt (stammen).

21. Verfahren nach einem der Ansprüche 1 bis 8, bei dem der Oligonucleotid-Primer ein nachweisbares Element an seinem 5'-Ende beinhaltet.

22. Verfahren nach Anspruch 21, bei dem das Trennen ein Abscheiden von irgendwelchem (irgendwelchen) verlängerten Primer(n) und ein erneutes Suspendieren des (der) verlähgerten Primer(s) in einer Lösung, die der Bindung des Trenn-Elements (der Trenn-Elemente) an (ein) Molekül(e) mit Affinität für das (die) Trenn-Element(e) förderlich ist, aufweist.

23. Verfahren nach Anspruch 1, bei dem Schritt d) aufweist:
d) Bestimmen, ob die spezielle Polynucleotid-Sequenz mit dem daran gebundenen verlängerten Primer in der Fraktion vorhanden ist, durch Untersuchen der Fraktion auf den verlängerten Primer, wobei die Anwesenheit des verlängerten Primers anzeigt, daß sich das spezielle Nucleotid oder die spezielle Base an der bestimmten Position in der speziellen Polynucleotid-Sequenz befindet, und das Fehlen des verlängerten Primers anzeigt, daß sich das spezielle Nucleotid oder die spezielle Base nicht an der bestimmten Position in der speziellen Polynucleotid-Sequenz befindet.

24. Verfahren nach Anspruch 2, bei dem Schritt d) aufweist:
d) Bestimmen, ob irgendwelche der speziellen Polynucleotid-Sequenzen mit den daran gebundenen verlängerten Primem in der Fraktion (den Fraktionen) vorhanden sind, durch Untersuchen der Fraktion(en) auf den (die) verlängerten Primer, wobei die Anwesenheit eines verlängerten Primers anzeigt, daß sich ein spezielles Nucleotid oder eine spezielle Base an einer bestimmten Position in einer speziellen Polynucleotid-Sequenz befindet, und das Fehlen des (der) verlängerten Primer(s) in einer Fraktion(en) anzeigt, daß sich mindestens ein spezielles Nucleotid oder mindestens eine spezielle Base nicht an einer bestimmten Position in mindestens einer verschiedenen speziellen Polynucleotid-Sequenz befindet.

25. Verfahren nach Anspruch 3, bei dem Schritt d) aufweist:
d) Bestimmen, ob sich mindestens eine spezielle Polynucleotid-Sequenz, an die einer der verlängerten Primer gebunden ist, in der Fraktion befindet, durch Untersuchen der Fraktion auf alle der verlängerten Primer, wobei die Anwesenheit eines verlängerten Primers anzeigt, daß sich mindestens ein spezielles Nucleotid oder mindestens eine spezielle Base an einer bestimmten Position in einer speziellen Polynucleotid-Sequenz befindet, und das Fehlen aller der verlängerten Primer in der Fraktion anzeigt, daß sich mindestens ein spezielles Nucleotid oder mindestens eine spezielle Base nicht an einer bestimmten Position in irgendeiner der verschiedenen speziellen Polynucleotid-Sequenzen befindet.

## Revendications

1. Procédé pour détecter si un nucléotide ou base spécifique est sur une position particulière dans une séquence polynucléotidique spécifique dans un matériau, comprenant
a) l'exposition, dans des conditions d'hybridation, dudit matériau à une amorce oligonucléotidique ayant une séquence complémentaire d'une partie de la séquence polynucléotidiqué spécifique, où ladite amorce incorpore un élément de séparation ou un élément détectable sur l'extrémité 5' de ladite amorce et où ladite amorce se lie à une partie de ladite séquence polynucléotidique spécifique dans ledit matériau (i) immédiatement au voisinage de la position particulière ou (ii) non immédiatement au voisinage de la position particulière, en conséquence de quoi il y a un intron entre la position particulière et l'amorce fixée à la séquence polynucléotidique spécifique ;
b) l'extension de l'amorce fixée à la séquence polynucléotidique spécifique, à la condition que l'amorce fixée ne soit étendue que jusqu'audit nucléotide ou base spécifique et incorpore celui-ci, quand ledit nucléotide ou base spécifique est sur la position particulière dans la séquence polynucléotidique spécifique où l'amorce étendue a un élément détectable et/ou un élément de séparation sur une position complémentaire dudit nucléotide ou base spécifique, à la condition que ladite amorce étendue ait au moins un élément de séparation et au moins un élément détectable et, quand il y a un intron entre la position particulière et l'amorce fixée à la séquence polynucléotidique spécifique, à la condition supplémentaire que l'intron ne puisse pas être une séquence où des bases ou nucléotides complémentaires de l'intron et qui sont incorporés dans l'amorce étendue provoquent l'incorporation d'un élément détectable et/ou de séparation interférant ;
c) la séparation de toute amorce étendue dans une fraction où ladite fraction n'a pas d'élément détectable non incorporé dans ladite amorce étendue, à la condition que ladite séparation ne comprenne pas l'étape de digestion de la séquence polynucléotidique spécifique ayant ladite amorce étendue fixée à celle-ci ; et
d) la détermination du fait que ladite amorce étendue est ou non présente dans ladite fraction par analyse sur ladite fraction de ladite amorce étendue, où la présence de ladite amorce étendue indique que le nucléotide ou base spécifique est sur la position particulière dans la séquence polynucléotidique spécifique et l'absence de ladite amorce étendue indique que le nucléotide ou base spécifique n'est pas sur la position particulière dans la séquence polynucléotidique spécifique ;
dans lequel ladite extension comprend l'utilisation d'au moins un didésoxynucléotide, ledit didésoxynucléotide étant complémentaire dudit nucléotide ou base spécifique.

2. Procédé pour détecter si des nucléotides ou bases spécifiques identiques ou différents sont sur des positions particulières dans au moins deux séquences polynucléotidiques spécifiques différentes dans un matériau ayant une pluralité de séquences polynucléotidiques différentes, comprenant :
a) l'exposition, dans des conditions d'hybridation, dudit matériau à au moins deux amorces oligonucléotidiques différentes, chacune desdites amorces oligonucléotidiques différentes ayant une séquence complémentaire d'une partie de l'une desdites séquences polynucléotidiques spécifiques différentes, où :
chacune desdites amorces se lie à sa séquence polynucléotidique complémentaire lorsqu'elle est présente dans ledit matériau, chacune desdites amorces oligonucléotidiques se liant à une partie d'une séquence polynucléotidique spécifique différente de celle de la ou des autres amorces,
chacune desdites amorces se lie à sa séquence polynucléotidique spécifique complémentaire dans ledit matériau (i) immédiatement au voisinage de la position particulière ou (ii) non immédiatement au voisinage de la position particulière, en conséquence de quoi il y a un intron entre la position particulière et l'amorce fixée à la séquence polynucléotidique spécifique quand elle est présente dans ledit matériau, et
chacune desdites amorces incorpore un élément de séparation ou un élément détectable sur l'extrémité 5' de ladite amorce ;
b) l'extension desdites différentes amorces oligonucléotidiques fixées à leurs séquences polynucléotidiques complémentaires, à la condition que chacune des amorces fixées ne soit étendue que jusqu'à la position particulière, et incorpore celle-ci, quand ledit nucléotide ou base spécifique est sur la position particulière dans la séquence nucléotidique spécifique où chacune desdites amorces étendues a un élément détectable et/ou un élément de séparation sur une position complémentaire dudit nucléotide ou base spécifique, à la condition en outre que chacune desdites amorces étendues ait au moins un élément de séparation et au moins un élément détectable et, quand il y a des introns entre les positions particulières et les amorces fixées aux séquences polynucléotidiques spécifiques, à la condition supplémentaire que les introns ne puissent pas être des séquences où des bases ou nucléotides complémentaires des introns et qui sont incorporés dans la ou les amorces étendues provoquent l'incorporation d'un ou plusieurs éléments détectables et/ou de séparation interférants ;
c) la séparation de toutes amorces étendues qui ne se sont étendues que jusqu'à un nucléotide ou base spécifique sur une position particulière dans au moins une fraction où ladite fraction n'a pas d'élément détectable non incorporé dans ladite amorce étendue, à la condition que ladite séparation ne comprenne pas l'étape de digestion de la séquence polynucléotidique spécifique ayant ladite amorce étendue fixée à celle-ci ; et
d) la détermination du fait que n'importe lesquelles desdites amorces étendues sont ou non présentes dans ladite ou lesdites fractions par analyse sur ladite ou lesdites fractions de ladite ou desdites amorces étendues, où la présence d'une amorce étendue indique qu'un nucléotide ou base spécifique est sur une position particulière dans une séquence polynucléotidique spécifique et l'absence de ladite ou desdites amorces étendues dans une ou plusieurs fractions indique qu'au moins un nucléotide ou base spécifique n'est pas sur une position particulière dans au moins une séquence polynucléotidique spécifique différente ;
dans lequel ladite extension comprend l'utilisation d'au moins un didésoxynucléotide, ledit didésoxynucléotide étant complémentaire dudit nucléotide ou base spécifique.

3. Procédé de criblage pour détecter si des nucléotides ou bases spécifiques identiques ou différents sont sur des positions particulières dans au moins deux séquences polynucléotidiques spécifiques différentes dans un matériau ayant une pluralité de séquences polynucléotidiques différentes, comprenant :
a) l'exposition, dans des conditions d'hybridation, dudit matériau à au moins deux amorces oligonucléotidiques différentes, chacune desdites amorces oligonucléotidiques différentes ayant une séquence complémentaire d'une partie de l'une desdites séquences polynucléotidiques spécifiques différentes, où :
chacune desdites amorces se lie à sa séquence polynucléotidique complémentaire lorsqu'elle est présente dans ledit matériau, chacune desdites amorces oligonucléotidiques se liant à une partie d'une séquence polynucléotidique spécifique différente de celle de la ou des autres amorces,
chacune desdites amorces se lie à sa séquence polynucléotidique spécifique complémentaire dans ledit matériau (i) immédiatement au voisinage de la position particulière ou (ii) non immédiatement au voisinage de la position particulière, en conséquence de quoi il y a un intron entre la position particulière et l'amorce fixée à la séquence polynucléotidique spécifique quand elle est présente dans ledit matériau, et
chacune desdites amorces incorpore un élément de séparation ou un élément détectable sur l'extrémité 5' de ladite amorce ;
b) l'extension desdites différentes amorces oligonucléotidiques fixées à leurs séquences polynucléotidiques complémentaires, à la condition que chacune des amorces fixées ne soit étendue que jusqu'à la position particulière, et incorpore celle-ci, quand ledit nucléotide ou base spécifique est sur la position particulière dans la séquence nucléotidique spécifique où chacune desdites amorces étendues a un élément détectable et/ou un élément de séparation sur une position complémentaire dudit nucléotide ou base spécifique, à la condition en outre que chacune desdites amorces étendues ait au moins un élément de séparation et au moins un élément détectable et, quand il y a des introns entre les positions particulières et les amorces fixées aux séquences polynucléotidiques spécifiques, à la condition supplémentaire que les introns ne puissent pas être des séquences où des bases ou nucléotides complémentaires des introns et qui sont incorporés dans la ou les amorces étendues provoquent l'incorporation d'un ou plusieurs éléments détectables et/ou de séparation interférants ;
c) la séparation de toutes amorces étendues qui- ne se sont étendues que jusqu'à un nucléotide ou base spécifique sur une position particulière dans une fraction où ladite fraction n'a pas d'élément détectable non incorporé dans ladite amorce étendue, à la condition que ladite séparation ne comprenne pas l'étape de digestion des séquences polynucléotidiques spécifiques ayant lesdites amorces étendues fixées à celles-ci ; et
d) la détermination du fait qu'au moins l'une lesdites amorces étendues est ou non présente dans ladite fraction par analyse sur ladite fraction de toutes lesdites amorces étendues, où la présence d'une amorce étendue indique qu'au moins un nucléotide ou base spécifique est sur une position particulière dans une séquence polynucléotidique spécifique et l'absence de toutes lesdites amorces étendues dans ladite fraction indique qu'au moins un nucléotide ou base spécifique n'est pas sur une position particulière dans l'une quelconque des séquences polynucléotidiques spécifiques différentes ;
dans lequel ladite extension comprend l'utilisation d'au moins un didésoxynucléotide, ledit didésoxynucléotide étant complémentaire dudit nucléotide ou base spécifique.

4. Procédé selon l'une quelconque des revendications 1 à 3, dans lequel ladite amorce se lie à une partie de ladite séquence polynucléotidique spécifique dans ledit matériau immédiatement au voisinage de la position particulière.

5. Procédé selon l'une quelconque des revendications 1 à 3, dans lequel ladite amorce se lie à une partie de ladite séquence polynucléotidique dans ledit matériau non immédiatement au voisinage de la position particulière, en conséquence de quoi il y a un intron entre la position particulière et l'amorce fixée à la séquence polynucléotidique spécifique.

6. Procédé selon la revendication 2 ou 3, dans lequel chacune desdites amorces incorpore un élément de séparation différent.

7. Procédé selon l'une quelconque des revendications 1 à 6, dans lequel ladite extension comprend l'utilisation de quatre nucléotides différents choisis dans le groupe constitué par dATP, dCTP, dGTP, dTTP, dUTP, dITP, ddATP, ddCTP, ddGTP, ddITP et ddTTP, au moins l'un desdits nucléotides étant choisi dans le groupe constitué par ddATP, ddCTP, ddGTP, ddITP et ddTTP, lesdits nucléotides incorporant un élément détectable et/ou un élément de séparation.

8. Procédé selon l'une quelconque des revendications 1 à 6, dans lequel ladite extension comprend l'utilisation d'un nucléotide choisi dans l'ensemble constitué par ddITP, ddATP, ddCTP, ddGTP et ddTTP, ledit nucléotide incorporant un élément détectable et/ou un élément de séparation.

9. Procédé selon la revendication 7 ou 8, dans lequel au moins l'un desdits nucléotides comprend un élément détectable.

10. Procédé selon la revendication 7 ou 8, dans lequel au moins l'un desdits nucléotides comprend un élément de séparation.

11. Procédé selon la revendication 7 ou 8, dans lequel au moins l'un desdits nucléotides comprend des éléments de séparation et détectable.

12. Procédé selon l'une quelconque des revendications 1 à 8, dans lequel ladite étape de séparation comprend : la mise en contact de toute amorce étendue avec une molécule présentant une affinité pour ledit élément de séparation ; et la séparation de ladite ou desdites amorces étendues mises en contact pour fournir ladite fraction où ledit élément de séparation et ladite molécule sont choisis dans le groupe indiqué comme suit :
| Elément de séparation | | Molécule présentant une affinité pour l'élément de séparation |
|---|---|---|
| (a) | un ligand pour lequel il y a un récepteur ; | un récepteur pour le ligand ; |
| (b) | un récepteur ; | le ligand pour le récepteur ; |
| (c) | un antigène ; | un anticorps pour l'antigène ; |
| (d) | un anticorps pour un antigène | l'antigène ; |
| (e) | un anticorps anti-idiotypique ; | un anticorps pour l'anticorps anti-idiotypique ; |
| (f) | un anticorps pour un anticorps anti-idiotypique ; | un anticorps anti-idiotypique pour l'anticorps |
| (g) | un groupe hapténique ; | un anticorps pour le groupe hapténique ; |
| (h) | un anticorps pour un groupe hapténique ; | le groupe hapténique ; |
| (i) | une enzyme ; | un inhibiteur de fixation pour l'enzyme ; et |
| (j) | un inhibiteur de fixation pour une enzyme ; | l'enzyme. |

13. Procédé selon la revendication 1, dans lequel ladite étape de séparation comprend : la mise en contact de toute amorce étendue avec une molécule présentant une affinité pour l'élément de séparation, ladite molécule étant liée à un support qui facilite ladite séparation ; et la séparation de ladite amorce étendue mise en contact pour fournir ladite fraction.

14. Procédé selon la revendication 2 ou 3, dans lequel ladite étape de séparation comprend : la mise en contact de toutes les amorces étendues avec des molécules présentant une affinité pour les éléments de séparation, lesdites molécules étant liées à des supports qui facilitent ladite séparation ; et la séparation desdites amorces étendues mises en contact pour fournir ladite fraction.

15. Procédé selon la revendication 13 ou 14, dans lequel ledit ou lesdits éléments de séparation et ladite ou lesdites molécules sont choisis dans le groupe indiqué comme suit :
| Elément de séparation | | Molécule présentant une affinité pour l'élément de séparation |
|---|---|---|
| (a) | un ligand pour lequel il y a un récepteur | un récepteur pour le ligand spécifique ; |
| (b) | un récepteur spécifique ; | le ligand pour le récepteur spécifique ; |
| (c) | un antigène ; | un anticorps spécifique pour l'antigène ; |
| (d) | un anticorps spécifique pour un antigène ; | l'antigène ; |
| (e) | un anticorps anti-idiotypique ; | un anticorps spécifique pour l'anticorps anti-idiotypique ; |
| (f) | un anticorps pour un anticorps anti-idiotypique ; | un anticorps anti-idiotypique spécifique pour l'anticorps |
| (g) | un groupe hapténique ; | un anticorps spécifique pour le groupe hapténique ; |
| (h) | un anticorps spécifique pour un groupe hapténique ; | le groupe hapténique ; |
| (i) | une enzyme ; | un inhibiteur de fixation étroite pour l'enzyme ; et |
| (j) | un inhibiteur de fixation étroite pour une enzyme ; | l'enzyme. |

16. Procédé selon la revendication 13, dans lequel ledit support est une bandelette de test.

17. Procédé selon la revendication 14 ou 15, dans lequel lesdits supports sont des bandelettes de test.

18. Procédé selon l'une quelconque des revendications 1 à 17, dans lequel la ou les séquences polynucléotidiques spécifiques sont une séquence d'ADN.

19. Procédé selon l'une quelconque des revendications 1 à 17, dans lequel la ou les séquences polynucléotidiques spécifiques sont une séquence d'ARN.

20. Procédé selon l'une quelconque des revendications 1 à 17, dans lequel la ou les séquences polynucléotidiques spécifiques proviennent d'un organisme infectieux ou provoquant une maladie.

21. Procédé selon l'une quelconque des revendications 1 à 8, dans lequel l'amorce oligonucléotidique incorpore un élément détectable sur son extrémité 5'.

22. Procédé selon la revendication 21, dans lequel ladite étape de séparation comprend la précipitation de toutes les amorces étendues et la remise en suspension de ladite ou desdites amorces étendues dans une solution qui contribue à la fixation dudit ou desdits éléments de séparation à une ou plusieurs molécules présentant une affinité pour ledit ou lesdits éléments de séparation.

23. Procédé selon la revendication 1, dans lequel l'étape d) comprend :
d) la détermination du fait que ladite séquence polynucléotidique spécifique ayant ladite amorce étendue fixée à celle-ci est ou non présente dans ladite fraction par analyse sur ladite fraction de ladite amorce étendue, où la présence de ladite amorce étendue indique que le nucléotide ou base spécifique est sur la position particulière dans la séquence polynucléotidique spécifique et l'absence de ladite amorce étendue indique que le nucléotide ou base spécifique n'est pas sur la position particulière dans la séquence polynucléotidique spécifique.

24. Procédé selon la revendication 2, dans lequel l'étape d) comprend :
d) la détermination du fait que n'importe lesquelles des séquences polynucléotidiques spécifiques ayant lesdites amorces étendues fixées à celles-ci sont ou non présentes dans ladite ou lesdites fractions par analyse sur ladite ou lesdites fractions de ladite ou desdites amorces étendues, où la présence d'une amorce étendue indique qu'un nucléotide ou base spécifique est sur une position particulière dans une séquence polynucléotidique spécifique et l'absence de ladite ou desdites amorces étendues dans une ou plusieurs fractions indique qu'au moins un nucléotide ou base spécifique n'est pas sur une position particulière dans au moins une séquence polynucléotidique spécifique différente.

25. Procédé selon la revendication 3, dans lequel l'étape d) comprend :
d) la détermination du fait qu'au moins une séquence polynucléotidique spécifique ayant l'une desdites amorces étendues fixée à celle-ci est ou non présente dans ladite fraction par analyse sur ladite fraction de toutes lesdites amorces étendues, où la présence d'une amorce étendue indique qu'au moins un nucléotide ou base spécifique est sur une position particulière dans une séquence polynucléotidique spécifique et l'absence de toutes lesdites amorces étendues dans ladite fraction indique qu'au moins un nucléotide ou base spécifique n'est pas sur une position particulière dans l'une quelconque des séquences polynucléotidiques spécifiques différentes.
